# EUROPEAN PATENT APPLICATION

(11) **EP 4 670 812 A1**
(43) Date of publication of application: **31.12.2025**
(21) Application number: 24185083.3
(22) Date of filing: 27.06.2024
(51) Int. Cl.: B01D 15/12, B01D 15/16, B01D 15/36, B01D 15/42, C12N 7/00, C12N 15/86, G01N 30/96

(54) **FLOW-THROUGH POLISHING OF VIRAL CAPSIDS**

(71) Applicant: Sartorius Stedim Biotech GmbH, 37079 Göttingen (DE)
(72) Inventor: Meierrieks, Frederik, 37079 Göttingen (DE); Graf, Benjamin, 37079 Göttingen (DE); Pickl, Andreas, 37079 Göttingen (DE); Pflanz, Karl, 37079 Göttingen (DE)
(74) Representative: Cohausz & Florack

(57) **Abstract**

The invention relates to a method of separating empty viral capsids and full viral capsids, the method comprising: providing a viral capsid preparation comprising the empty viral capsids and the full viral capsids, wherein the viral capsid preparation comprises a first salt concentration; contacting the viral capsid preparation with a stationary phase surface allowing binding of the full viral capsids to the stationary phase surface, wherein the empty viral capsids at least partially do not bind to the stationary phase surface; and conducting a one-step and/or linear elution in which the full capsids are eluted by contacting the stationary phase obtained in step (ii) with an elution solution comprising a second salt concentration, wherein the first salt concentration is lower than the second salt concentration.

## Description

### FIELD OF THE INVENTION

The invention pertains to a method for separation or depletion of empty viral capsids from full viral capsids. The invention provides a method of separating empty viral capsids and full viral capsids, the method comprising: (i) providing a viral capsid preparation comprising the empty viral capsids and the full viral capsids, wherein the viral capsid preparation comprises a first salt concentration; (ii) contacting the viral capsid preparation with a stationary phase surface allowing binding of the full viral capsids to the stationary phase surface, wherein the empty viral capsids at least partially do not bind to the stationary phase surface; and (iii) conducting an one-step elution and/or linear salt gradient elution in which the full capsids are eluted by contacting the stationary phase obtained in step (ii) with an elution solution comprising a second salt concentration, wherein the first salt concentration is lower than the second salt concentration.

### BACKGROUND OF THE INVENTION

Viruses find application in a variety of therapeutic areas, including vaccines, oncolytic viruses, and as vectors in gene therapeutic treatments. Among these, adeno-associated virus (AAV) vectors have emerged as particularly promising for in vivo gene therapy due to their non-pathogenicity, low immunogenicity, ease of production, and transgene expression in various quiescent cell types. To date, the U.S. Food and Drug Administration (FDA) has granted approval to five AAV-based gene therapies: Elevidiys (Sarepta), Hemgenix (CSL), Luxturna (Spark), Roctavian (BioMarin), and Zolgensma (Novartis).

Discovered in 1965 within adenovirus (AdV) preparations, AAV belongs to the Parvoviridae family and the genus of Dependoparvovirus. Its replication is dependent on the presence of a helper virus, such as an AdV. 10 AAV is a non-enveloped, 4.7 kb single-stranded DNA (ssDNA) virus with an icosahedral capsid of 25 nm in diameter. The AAV capsid consists of 60 subunits of the three viral proteins (VP) VP1, VP2, and VP3, which are organized in an icosahedral symmetry in a ratio of 1:1:10. To date, 13 different human serotypes have been identified. These serotypes are characterized by variations in their capsid proteins and have specific tropisms for different types of cells.

AAV are commonly produced by transfection of human embryonic kidney (HEK) 293 cells. Alternative production methods include the transfection of HeLa cells, stable cell lines, or the use of a baculovirus-Sf9 cell system. The purification of AAV typically involves multiple steps, beginning with cell lysis and nuclease treatment, followed by clarification through filtration. This is followed by a chromatographic capture step using either immunoaffinity chromatography (IAC) or ion exchange chromatography (IEC). Final steps include a polishing step for the enrichment of full AAV particles, concentration and formulation using tangential flow filtration (TFF), and sterile filtration.

Regardless of the manufacturing process, the encapsulation of DNA into the capsids during AAV production results in particles containing an intact genome (full), particles devoid of genomic content (empty).

Although empty AAV particles have the same capsid structure as full AAV particles, they lack the therapeutic gene of interest (GOI) and are therefore not functionally relevant for the intended therapeutic effect. Consequently, empty AAV particles are considered as product-related impurities, while their precise role for gene therapeutic treatments remains unclear.

It is contemplated that empty capsids may serve as decoys for naturally occurring neutralizing anti-AAV antibodies, potentially enhancing the efficacy of a gene therapy drug. On the other hand, they may compete with full AAV particles for receptor binding sites, internalization, and intracellular cargo, which could reduce therapeutic efficacy. Beyond efficacy concerns, empty AAV particles may also affect the safety profile of AAV-based gene therapies by increasing the risk of immunogenicity. For instance, Nathwani et al. observed that an excess of empty AAV8 particles could induce AAV8-capsid-specific T-cell responses, resulting in low-grade hepatotoxicity and partial loss of transgene expression (Nathwani, A.C., Tuddenham, E.G.D., RangaraJan, S., Rosales, C., McIntosh, J., Linch, D.C., Chowdary, P., Riddell, A., Pie, A.J., and Harrington, C., et al. (2011). Adenovirus-associated virus vector-mediated gene transfer in hemophilia B. The New England Journal of medicine 365, 2357-2365). In addition, Mingozzi et al. reported anti-capsid T-cell responses following ocular administration of AAV2 (Mingozzi, F., Maus, M.V., Hui, D.J., Sabatino, D.E., Murphy, S.L., Rasko, J.E.J., Ragni, M.V., Manno, C.S., Sommer, J., and Jiang, H., et al. (2007). CD8(+) T-cell responses to adeno-associated virus capsid in humans. Nature medicine 13, 419-422).

Therefore, in addition to the potency of an AAV gene therapy drug, it is crucial to ensure its safety profile through a manufacturing process that results in a final product with a low level of empty particles.

The separation of full and empty AAV particles is challenging due to their identical capsid structure, which renders immunoaffinity chromatography ineffective for separation. Separation of empty full AAV can be achieved by various methods, such as size exclusion chromatography (SEC), density gradient centrifugation, or anion exchange chromatography (AEC). Among these, density gradient centrifugation provides a serotype-independent approach to separate full and empty AAV particles based on their sedimentation characteristics. However, density gradient centrifugation faces scalability challenges, is prone to operator error, and requires expensive equipment.

AEC is a method that exploits the interaction of the stationary phase with the surface charge of the viral particles. Empty AAV have an isoelectric point (pI) of about 6.3, while full AAV have a pI of about 5.9 due to their genomic payload. This marginal difference in pI of about 0.4 enables the precise separation of full AAV from their empty counterparts. Unlike density gradient centrifugation, AEC provides a scalable solution for separating empty from full AAV particles. Chromatographic separation is typically achieved using linear elution gradients or isocratic elution. A variety of chromatographic matrices, including resins, membranes, or monoliths, can be used for AEC-based separation of empty and full AAV. Traditionally, AEC-based methods for separating empty from full AAV depend on the use of fast protein liquid chromatography (FPLC) systems.

The purification of AAV, in particular the separation of genome-containing from empty AAV capsids, is therefore usually time-consuming and requires expensive equipment. Especially for academic research, the separation of full and empty AAV remains a bottleneck.

There is therefore an unmet need for rapid and easy-to-perform methods for separating full viral capsids from empty viral capsids.

### SUMMARY OF THE INVENTION

Against the aforementioned background, it is an object of the present invention to provide improved methods for separating full viral capsids from empty viral capsids. Is also an object of the present invention to provide less time-consuming methods for separating full viral capsids from empty viral capsids, which can be especially useful in research and development of novel gene therapies. A further object of the present invention is to provide easy-to-use methods for separating full viral capsids from empty viral capsids.

These objects are achieved by the invention set forth in the claims and embodiments explained in more detail below.

The invention provides a method of separating empty viral capsids and full viral capsids, the method comprises: (i) providing a viral capsid preparation comprising the empty viral capsids and the full viral capsids, wherein the viral capsid preparation comprises a first salt concentration; (ii) contacting the viral capsid preparation with a stationary phase surface allowing binding of the full viral capsids to the stationary phase surface, wherein the empty viral capsids at least partially do not bind to the stationary phase surface; and (iii) conducting a one-step elution and/or linear salt gradient elution in which the full capsids are eluted by contacting the stationary phase obtained in step (ii) with an elution solution comprising a second salt concentration, wherein the first salt concentration is lower than the second salt concentration.

The invention further provides to a kit comprising: a first container comprising a preparation solution with a first salt concentration, and a second container comprising an elution solution with a second salt concentration, wherein the first salt concentration is lower than the second salt concentration.

The inventors found a novel easy-to-use flow-through polishing method for separating full and empty viral capsids. By using this method, empty capsids do not bind to the stationary phase surface, while full capsids are retained and can be subsequently eluted by using a buffer of increased ionic strength. This method beneficially allows the use of syringes instead of an FPLC system to separate full empty viral capsids from empty viral capsids.

The novel method disclosed herein eliminates the need for a chromatography system. Moreover, the method disclosed herein was shown to preserve the functional potency and structural integrity of the viral capsids. The method can be conducted in a manual syringe-based system, allowing for an easy and fast separation of full and empty viral capsids.

This novel flow-through polishing method demonstrates the feasibility of separating full and empty viral capsids without complex linear or stepped gradient elution and the necessity of specialized equipment. The method provided by the invention therefore provides a rapid and easy-to-perform platform for polishing multiple viral vector preparations, addressing a critical aspect in the research and development of novel gene therapies.

### DETAILED DESCRIPTION

Although certain embodiments of the present invention are described in detail below, it is to be understood that this invention is not limited to the particular embodiments, methodologies, protocols and reagents described herein as these may vary within the scope set by the claims. It is also to be understood that terminology used herein is for the purpose of describing particular embodiments only, and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The above objects are achieved by the following embodiments in accordance with the invention:
1. A method of separating empty viral capsids and full viral capsids, the method comprising:
   (i) providing a viral capsid preparation comprising the empty viral capsids and the full viral capsids, wherein the viral capsid preparation comprises a first salt concentration;
   (ii) contacting the viral capsid preparation with a stationary phase surface allowing binding of the full viral capsids to the stationary phase surface, wherein the empty viral capsids at least partially do not bind to the stationary phase surface; and
   (iii) conducting a one-step elution and/or linear salt gradient elution in which the full capsids are eluted by contacting the stationary phase obtained in step (ii) with an elution solution comprising a second salt concentration.
   wherein the first salt concentration is lower than the second salt concentration.
2. The method according to embodiment 1, wherein the first salt concentration is at least about 0.5 mM or at least about 1 mM lower than the second salt concentration.
3. The method according to any of the preceding embodiments, wherein the method comprises an optional washing step.
4. The method according to any of the preceding embodiments, wherein the one-step elution is an isocratic elution.
5. The method according to any of the preceding embodiments, wherein the pH of the viral capsid preparation and the pH of the elution solution differ about 1 pH unit or less, about 0.9 pH units or less, about 0.8 pH units or less, preferably about 0.7 pH units or less, preferably about 0.6 pH units or less, preferably about 0.5 pH units or less, preferably about 0.4 pH units or less, preferably about 0.3 pH units or less, more preferably about 0.2 pH units or less, most preferably about 0.1 pH units or less.
6. The method according to any of the preceding embodiments, wherein the pH of the viral capsid preparation and the pH of the elution solution are within a range of about 6 to about 10, preferably about 7 to about 9, preferably about 8 to about 9.
7. The method according to any of the preceding embodiments, wherein the first and second salt concentration is a first and second chaotropic salt concentration.
8. The method according to any of the preceding embodiments, wherein the salt comprises a monovalent or divalent cation.
9. The method according to any of the preceding embodiments, wherein the salt comprises Mg²⁺, Ca⁺, or Na⁺ or any combination thereof.
10. The method according to any of the preceding embodiments, wherein the salt comprises acetate, chloride, or sulfate or any combination thereof.
11. The method according to any of the preceding embodiments, wherein the salt comprises or is selected from magnesium sulfate, sodium sulfate, calcium sulfate, magnesium chloride, sodium chloride, calcium chloride, magnesium acetate, sodium acetate, calcium acetate, or any combination thereof.
12. The method according to any of the preceding embodiments, wherein the salt comprises or is selected from magnesium sulfate, sodium sulfate, calcium sulfate, magnesium chloride, calcium chloride, magnesium acetate, sodium acetate, calcium acetate, or any combination thereof.
13. The method according to embodiment 11 or 12, wherein the salt comprises or is selected from magnesium sulfate, sodium sulfate, or calcium sulfate or any combination thereof.
14. The method according to embodiment 11, wherein the salt comprises or is selected from magnesium chloride, sodium chloride, or calcium chloride or any combination thereof.
15. The method according to embodiment 11 or 12 wherein the salt comprises or is selected from magnesium acetate, sodium acetate, or calcium acetate or any combination thereof.
16. The method according to any of the preceding embodiments, wherein the viral capsid preparation comprises a first magnesium chloride concentration.
17. The method according to any of the preceding embodiments, wherein the elution solution comprises a second magnesium chloride concentration.
18. The method according to any of the preceding embodiments, wherein the full viral capsids and empty viral capsids are *Parvoviridae* capsids.
19. The method according to any of the preceding embodiments, wherein the full viral capsids and empty viral capsids are *Dependoparvovirus* capsids.
20. The method according to any of the preceding embodiments, wherein the full viral capsids and empty viral capsids are *Adeno-associated viruses* (AAV) capsids.
21. The method according to embodiment 20, wherein the AAV has a serotype selected from the group consisting of AAV1, AAV2, AAV3, AAV4, AAV5, AAV6, AAV7, AAV8, AAV9, AAV10, AAV1 1, AAV12, AAV13, a synthetic recombinant serotype, a recombinant hybrid serotype, and combinations thereof.
22. The method according to any of the preceding embodiments, wherein the method comprises one or more contacting steps (ii).
23. The method according to any of the preceding embodiments, wherein the method comprises a single contacting step (ii).
24. The method according to any of the preceding embodiments, wherein a flow-through fluid comprising unbound empty capsids is obtained in step (ii).
25. The method according to any of the preceding embodiments, wherein the viral capsid preparation is an aqueous preparation, preferably a buffered aqueous preparation.
26. The method according to any of the preceding embodiments, wherein the elution solution is an aqueous solution, preferably a buffered aqueous solution.
27. The method according to any of the preceding embodiments, wherein the viral capsid preparation and/or elution solution comprises a buffering component controlling the pH at about 6 to about 10, preferably about 7 to about 9, preferably about 8 to about 9, such as Tris, preferably about 5 mM to 200 mM Tris, preferably about 10 to about 100 mM Tris.
28. The method according to any of the preceding embodiments, wherein the first salt concentration is in the range of 0.01 to 100 mM.
29. The method according to any of the preceding embodiments, wherein the second salt concentration is in the range of 0.1 to 140 mM.
30. The method according to any of the preceding embodiments, wherein the full viral capsids and empty viral capsids are AAV2 capsids.
31. The method according to embodiment 30, wherein the first salt concentration is in the range of about 0.01 to about 10 mM, about 0.02 to about 8 mM, about 0.03 to about 7 mM, about 0.04 to about 6 mM, about 0.05 to about 5 mM, about 0.06 to about 4 mM, about 0.07 to about 3 mM, or about 0.08 to about 2 mM.
32. The method according to embodiment 30 or 31, wherein the second salt concentration is in the range of about 0.1 to about 15 mM, about 0.5 to about 13 mM, about 1 to about 11 mM, about 1.5 to about 9 mM, about 2 to about 7 mM, about 2.5 to about 5 mM, or about 3 to about 4 mM.
33. The method according to any of embodiments 1-29, wherein the full viral capsids and empty viral capsids are AAV5 capsids.
34. The method according to embodiment 33, wherein the first salt concentration is in the range of about 0.5 to about 100 mM, about 1 to about 80 mM, about 2 to about 60 mM, about 3 to about 40 mM, about 4 to about 30 mM, about 5 to about 25 mM, about 6 to about 20 mM, or about 7 to about 15 mM.
35. The method according to embodiment 33 or 34, wherein the second salt concentration is in the range of about 1 to about 140 mM, about 3 to about 120 mM, about 4 to about 100 mM, about 5 to about 80 mM, about 8 to about 60 mM, about 9 to about 40 mM, or about 10 to about 30 mM.
36. The method according to any of embodiments 1-29, wherein the full viral capsids and empty viral capsids are AAV8 capsids.
37. The method according to embodiment 36, wherein the first salt concentration is in the range of about 0.1 to about 100 mM, about 0.5 to about 80 mM, about 1 to about 60 mM, about 1.5 to about 40 mM, about 2 to about 30 mM, about 2.5 to about 20 mM, or about 3 to about 10 mM.
38. The method according to embodiment 36 or 37, wherein the second salt concentration is in the range of about 1 to about 140 mM, about 2 to about 120 mM, about 3 to about 100 mM, about 4 to about 80 mM, about 5 to about 60 mM, about 6 to about 40 mM, or about 7 to about 20 mM.
39. The method according to any of the preceding embodiments, wherein the viral capsid preparation is a purified crude cell harvest or a purified crude cell lysate.
40. The method according to any of the preceding embodiments, wherein the method further comprises the step of providing a crude cell harvest or crude cell lysate comprising the first salt concentration, and purifying the crude cell harvest or crude cell lysate to obtain the viral capsid preparation of step (i) (e.g., by steric exclusion chromatography).
41. The method according to any of the preceding embodiments, wherein the cells are HEK293 cells, HELA cells, CHO cells, insect cells, or derivatives thereof.
42. The method according to any of the preceding embodiments, wherein the method is conducted in a syringe-based system.
43. The method according to any of the preceding embodiments, wherein the stationary phase is a chromatographic stationary phase.
44. The method according to any of the preceding embodiments, wherein the stationary phase is characterized by a monolithic structure, membrane, or resin with a multimodal ligand that combines hydrogen bonding with a weak anion exchanger.
45. The method according to any of the preceding embodiments, wherein the stationary phase comprises a quaternary amine ligand and thus a strong anion exchanger.
46. A kit comprising:
   (i) a first container comprising a preparation solution with a first salt concentration
   (ii) a second container comprising an elution solution with a second salt concentration
   wherein the first salt concentration is lower than the second salt concentration.
47. The kit according to embodiment 46, further comprising a stationary phase surface allowing binding of the full viral capsids to the stationary phase surface.
48. The kit according to embodiment 46 or 47, further comprising a syringe-based system comprising the stationary phase surface.
49. The kit of any of embodiments 46-48 for use in the method of any of embodiments 1-45.
50. The kit according to any of embodiments 46-49, wherein the preparation and/or elution solution comprises a buffering component controlling the pH at about 6 to about 10, preferably about 7 to about 9, preferably about 8 to about 9, such as Tris, preferably about 5 mM to 200 mM Tris, preferably about 10 to about 100 mM Tris.
51. The kit according to any of embodiments 46-50, wherein the pH of the preparation solution and the pH of the elution solution differ about 1 pH unit or less, about 0.9 pH units or less, about 0.8 pH units or less, preferably about 0.7 pH units or less, preferably about 0.6 pH units or less, preferably about 0.5 pH units or less, preferably about 0.4 pH units or less, preferably about 0.3 pH units or less, more preferably about 0.2 pH units or less, most preferably about 0.1 pH units or less.
52. The kit according to any of embodiments 46-51, wherein the pH of the preparation solution and the pH of the elution solution are within a range of about 6 to about 10, preferably about 7 to about 9, preferably about 8 to about 9.
53. The kit according to any of embodiments 46-52, wherein the salt comprises or is selected from magnesium sulfate, sodium sulfate, calcium sulfate, magnesium chloride, sodium chloride, calcium chloride, magnesium acetate, sodium acetate, calcium acetate, or any combination thereof, preferably wherein the salt comprises or is selected from magnesium sulfate, sodium sulfate, calcium sulfate, magnesium chloride, calcium chloride, magnesium acetate, sodium acetate, calcium acetate, or any combination thereof.
54. The kit according to any of embodiments 46-53, wherein the salt comprises or is selected from magnesium sulfate, sodium sulfate, or calcium sulfate or any combination thereof.
55. The kit according to any of embodiments 46-53, wherein the salt comprises or is selected from magnesium chloride, sodium chloride, or calcium chloride or any combination thereof.
56. The kit according to any of embodiments 46-53, wherein the salt comprises or is selected from magnesium acetate, sodium acetate, or calcium acetate or any combination thereof.

### Definitions

The terms indicated for explanation of the invention have the following meaning, unless otherwise indicated in the description or the claims. Additional definitions are set forth throughout the detailed description.

Terms "a" and "an" and "the" and similar reference used in the context of describing the invention (especially in the context of the claims) are to be construed to cover both the singular and the plural, unless otherwise indicated herein or clearly contradicted by context.

The terms "about" or "approximately" as used herein denotes a range of ±10% of a reference value. For examples, "about 10" defines a range of 9 to 11. In general, those skilled in the art, familiar with the context, will appreciate the relevant degree of variance encompassed by "about" or "approximately" in that context.

As used herein, the term "adeno-associated virus" (AAV), includes but is not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

Affinity chromatography is a form of liquid chromatography that uses a biologically-related binding agent as the stationary phase. This technique can be used for the isolation and purification of specific targets by taking advantage of the selective and reversible binding which occurs in many biological interactions. Examples of theses interactions are those which occur between an antibody and antigen, enzyme and substrate, or hormone and receptor. Affinity chromatography makes use of these systems by immobilizing one of the pair of interacting agents onto a chromatographic support. The agent that is immobilized onto the support is known as the "affinity ligand" and provides a stationary phase surface with the ability to selectively retain the complementary target even when this target compound is present in a complex mixture.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

A "viral capsid" or "capsid" is a particle that comprises at least one viral capsid protein which (i) encapsidates a nucleic acid, e.g., a vector genome or a portion thereof, and/or (ii) forms a structure surrounding a core. An "empty capsid" or "empty viral capsid" is a capsid particle that includes at least one capsid protein and lacks a complete vector genome, e.g., lacks in whole or in part, a heterologous nucleic acid of interest flanked on either side by AAV ITRs, or lacks in whole or in part, another part of the vector genome. In the case of an empty capsid, as described herein, the core may be empty, or collapsed, or may contain only a portion of a vector genome or may contain a portion of the host cell DNA. In some embodiments, a viral capsid encapsidates a nucleic acid that is a vector genome and/or gene of interest. In some embodiments, a viral capsid encapsidates a nucleic acid that is not a vector genome or gene of interest, e.g., plasmid or host cell DNA, or a portion thereof.

The "contacting step" refers to the loading of a stationary phase surface to allow for binding of the full viral capsids to the stationary phase surface. In this step, the viral capsid preparation can be passed through the stationary phase system and therethrough applied to the stationary phase surface. The flowthrough fluid of the viral capsid preparation comprising unbound viral capsids can be collected.

A "full capsid" or "full viral capsid" is a capsid particle that comprises a complete vector genome, that is, a vector genome that preferably comprises a heterologous nucleic acid of interest flanked on both sides by AAV ITRs.

The term "equilibrate" or "equilibration" refers to a chemical conditioning step performed on the stationary phase surface and/or on the sample to create a specific chemical environment. Stationary phases are customarily conditioned by exposing them to a buffer that comprises the desired pH, salt composition, and salt concentration. Samples are customarily conditioned by titration of pH, by dilution to reduce the salt concentration, and/or by buffer exchange techniques including by chromatography, or by dialysis, or by diafiltration with tangential flow filtration membranes. For example, equilibration conditions can be set so the stationary phase binds full capsids, while the majority or all of the empty capsids fail to bind to the stationary phase during the contacting step (ii) of the method disclosed herein.

Unless expressly specified otherwise, the term "comprising" is used in the context of the present disclosure to indicate that further members may optionally be present in addition to the members of the list introduced by "comprising". It is, however, contemplated as specific embodiments of the present invention that each time the term "comprising" is used, this shall also encompass the possibility of no further members being present, i.e., for the purpose of such specific embodiments "comprising" can be understood as having the meaning of "consisting of'.

Digital Polymerase Chain Reaction (dPCR) is a method for the absolute quantification of target nucleic acids. dPCR is well known to the skilled person and e.g., described in Quan, Phenix-Lan et al. "dPCR: A Technology Review." Sensors (Basel, Switzerland) vol. 18,4 1271. 20 Apr. 2018, doi:10.3390/s18041271.

Herein, the term "DNA" relates to a nucleic acid molecule which is entirely or at least substantially composed of deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. DNA may be recombinant DNA and may be obtained by cloning of a nucleic acid, in particular cDNA. The cDNA may be obtained by reverse transcription of RNA.

The term "elution" or "eluted" refers to a process of changing the chemical environment in which the stationary phase resides to cause dissociation of the interaction between the stationary phase surface and the viral capsids that remained bound after the contacting step and optional washing step.

As used herein, the term "gene" refers to the segment of a DNA molecule that codes for a polypeptide chain (e.g., the coding region). In some embodiments, a gene is positioned by regions immediately preceding, following, and/or intervening the coding region that are involved in producing the polypeptide chain (e.g., regulatory elements such as a promoter, enhancer, polyadenylation sequence, 5'-untranslated region (5'UTR), 3'-untranslated region (3'UTR), or intron).

A "gradient elution" is a mode of chromatographic separation wherein the concentration of one or more salts in the elution solution and/or the pH of the elution solution that is applied to the stationary phase surface is gradually changed (increased or decreased) during the elution step.

An "inverted terminal repeat" (abbreviated "ITR") is a symmetrical nucleic acid sequences in the genome of adeno-associated viruses required for efficient replication. ITR sequences are located at each end of the AAV DNA genome. The ITRs serve as the origins of replication for viral DNA synthesis and are essential cis components for generating AAV integrating vectors.

The use of the term "include," `includes," 'including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

The "isoelectric point" (pI) is a special pH value at which the net charge of protein or virus is zero. Proteins carry positive charge at a pH below the pI, while proteins carry negative charge at a pH above the pI.

"Fast protein liquid chromatography" (FPLC) is a well-known form of high-performance chromatography that takes advantage of the high resolution made possible by small-diameter stationary phases. It was originally developed for proteins and features high loading capacity, biocompatible aqueous buffer systems, fast flow rates, and availability of stationary phases in most common chromatography modes (e.g., ion exchange, gel filtration, reversed phase, and affinity). The system usually makes reproducible separation possible by incorporating a high level of automation including autosamplers, gradient program control, and peak collection. In addition to proteins, the method is applicable to other kinds of biological samples including oligonucleotides and plasmids. The most common type of FPLC experiment is anion exchange of proteins.

As used herein, by "obtaining" or "to obtain" with respect to a "fraction", "eluent", "flow through", or "wash fraction" - e.g., an eluent after a step of conducting an elution by contacting an elution solution to a stationary phase surface - it is meant that the fraction or eluent is generated during and until the end of that step. A fraction that is "obtained" or "generated" may or may not be collected.

The terms "polypeptide," "peptide," and "protein" are used interchangeably herein to refer to polymers of amino acids.

The term "recombinant" when used in the context of a virus or serotype is a virus or serotype having nucleotide or amino acid sequences that are not naturally joined together and can be made by artificially combining two otherwise separated segments of sequence through human intervention. This artificial combination is often accomplished by chemical synthesis or, more commonly, by the artificial manipulation of isolated segments of nucleic acids, for example, by genetic engineering techniques. Recombinant serotypes express or comprise vectors comprising an amplified or assembled polynucleotide, which can be used to transform or transfect a suitable host cell.

A "recombinant hybrid serotype" is a serotype which comprise nucleotide or amino acid sequences (such as capsid sequences) which naturally occur in two distinct serotypes. Serology is commonly defined as the inability of an antibody that is reactive to one serotype in neutralizing another serotype.

A "synthetic recombinant serotype" is a serotype (such as an AAV serotype) which comprise nucleotide or amino acid sequences (such as capsid sequences) which do not naturally occur in any natural serotype (such as an AAV serotype). These sequences can be produced by chemical synthesis or genetic engineering techniques.

"Recombinant AAV" (rAAV) and "AAV" are used interchangeably throughout the application.

"Tangential flow filtration" (TFF) is a way of removing impurities from large-volume samples. It involves using a filter to separate or isolate contaminant particles. Unlike dead-end filtration, in TFF fluid is circulated across, rather than forced through, a filter, resulting in a pressure differential that keeps contaminants from re-mixing with newly purified samples.

As used herein, a "transgene" is a nucleic acid that is introduced into the cell, including but not limited to genes or nucleic acid having sequences which are not normally present in AAV, genes which are present but not normally transcribed and translated ("expressed") in an AAV genome, or any other gene or nucleic acid which one desires to position between the ITR sequences. A transgene may include one or more transcriptional regulatory sequences and any other nucleic acid, such as introns, that may be necessary for optimal expression of a selected nucleic acid. A transgene can comprise coding or non-coding sequences.

As used herein, the terms "virus vector," "viral vector," and "gene delivery vector" refer to a virus particle that functions as a nucleic acid delivery vehicle, and which comprises a nucleic acid molecule (such as DNA) packaged within a viral capsid. Exemplary virus vectors include adeno-associated virus (AAV) vectors.

As used herein, the term "transduction efficiency" or "potency" refers to the ability of a viral vector or viral particle, preferably an rAAV particle, to transduce a target cell and express a transgene in the target cell. Transduction efficiency can be represented in Transducing units (e.g., TU per volume). Transducing unit as used herein refers to the number of functional viral vectors or viral particles, preferably rAAV particles, in a solution that are capable of transducing a target cell and expressing a transgene in the target cell. The skilled person knows how to determine the transduction efficiency of a viral vector or a viral particle.

A "viral capsid preparation" is a product that results from a method of manufacturing recombinant viral vectors such as AAV vectors in a host cell (e.g., in a mammalian cell or an insect cell). A viral capsid preparation includes a mixture of full viral capsids and empty viral capsids. In some embodiments, a viral capsid preparation has been subjected to one or more downstream operations after initial upstream operations, e.g., lysis treatment, nuclease treatment, filtration to remove host-cell impurities, and/or affinity purification using ligands that bind AAV capsids, as well known to those of skill in the art.

A "washing step" or "rinsing step" refers to a process of exposing the stationary phase surface to a buffer for the purpose of displacing unbound empty capsid from the stationary phase surface. In some embodiments, the washing solution has the same formulation as the viral capsid preparation but lacks the full and empty viral capsids. In some embodiments, the washing solution has the same formulation as the equilibration buffer. In some embodiments, the washing solution may displace weakly bound contaminants (e.g., empty capsids) from the stationary phase surface so that they can be removed before the full capsids are eluted. Or there may be more than one washing step where the first washing step employs conditions identical to the equilibration buffer but the second washing step employs conditions that displace a subset of weakly bound contaminants from the stationary phase surface.

Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

References to "one embodiment," "an embodiment," "example embodiment," "some embodiments," "certain embodiments," "various embodiments," etc., indicate that the embodiment(s) of the disclosed technology so described may include a particular feature, structure, or characteristic, but not every embodiment necessarily includes the particular feature, structure, or characteristic.

All patents, patent applications, and other publications cited in this application are incorporated by reference in the entirety for all purposes.

The invention provides a method of separating empty viral capsids and full viral capsids, the method comprising:
(i) providing a viral capsid preparation comprising the empty viral capsids and the full viral capsids, wherein the viral capsid preparation comprises a first salt concentration;
(ii) contacting the viral capsid preparation with a stationary phase surface allowing binding of the full viral capsids to the stationary phase surface, wherein the empty viral capsids at least partially do not bind to the stationary phase surface; and
(iii)conducting a one-step elution and/or linear salt gradient elution in which the full capsids are eluted by contacting the stationary phase obtained in step (ii) with an elution solution comprising a second salt concentration.
wherein the first salt concentration is lower than the second salt concentration.

The invention beneficially makes use of the different pI of full and empty viral capsids to provide an easy-to-use and rapid separation method. The method beneficially comprises a contacting step (loading step) in which the full capsids of the viral capsid preparation are applied to the stationary phase surface, while the empty capsids at least partially do not bind to the stationary phase surface. The empty capsids can be collected in the flowthrough fluid. A one-step elution is sufficient to elute and collect the bound full viral capsids. The method does not require complex FPLC-systems or complex elution steps since a syringe-base system and a single elution step suffices for separation. The method further allows mild separation conditions, e.g., under constant pH, allowing the full viral capsids to maintain their integrity and stability. The invention is therefore particularly useful for the research and development of gene therapies.

The stationary phase is the media through which the viral capsid preparation (i.e., mobile phase) passes through such as a membrane adsorber or a monolithic media. According to some embodiments, the stationary phase comprises at least one material selected from the group composed of natural or synthetic fibers, (polymer) membranes, porous (polymer) particles, monolithic solid supports, polymer gels, films, nonwovens and wovens. In some preferred embodiments, the stationary phase is a monolithic solid support. In some embodiments, an agent (i.e., an "affinity ligand") is immobilized onto the support and provides a stationary phase surface with the ability to selectively bind full viral capsids. Exemplary stationary phase systems suitable for the method disclosed herein are anion exchange resins such as CIMmultus QA^{®}, CIMmultus PrimaS^{®}, and Sartobind Q^{®} Membrane Adsorbers (all available from Sartorius).

In some embodiments, the first salt concentration is at least about 0.5 mM or at least about 1 mM lower than the second salt concentration. The first salt concentration can be at least about 0.6 mM, at least about 0.7 mM, at least about 0.8 mM, at least about 0.9 mM, at least about 1.0 mM, at least about 1.5 mM, at least about 2.0 mM, at least about 3.0 mM, at least about 4.0 mM, at least about 5.0 mM, at least about 6.0 mM, at least about 7.0 mM, at least about 8.0 mM, at least about 9.0 mM, or at least about 10.0 mM lower than the second salt concentration. For example, the first salt concentration can be about 0.6 mM, about 0.7 mM, about 0.8 mM, about 0.9 mM, about 1.0 mM, about 1.5 mM, about 2.0 mM, about 3.0 mM, about 4.0 mM, about 5.0 mM, about 6.0 mM, about 7.0 mM, about 8.0 mM, about 9.0 mM, or about 10.0 mM lower than the second salt concentration.

In some particularly preferred embodiments, the method disclosed herein does not comprise a linear gradient elution, preferably not a linear salt gradient elution and/or linear pH gradient elution, more preferably not a linear salt gradient and linear pH gradient elution. In some particularly preferred embodiments, the method disclosed herein does not comprise a pH gradient elution.

The method may comprise: (i) providing a viral capsid preparation comprising the empty viral capsids and the full viral capsids, wherein the viral capsid preparation comprises a first salt concentration; (ii) contacting a volume of the viral capsid preparation with a stationary phase surface in an anion exchange system and passing the volume of viral capsid preparation through the anion exchange system to generate a flowthrough comprising empty viral capsids; and (iii) conducting an one-step elution and/or linear salt gradient elution step by contacting the stationary phase surface with an elution solution comprising a second salt concentration and passing the elution solution through the anion exchange system to generate an eluent comprising full viral capsids.

The method may preferably comprise: (i) providing a viral capsid preparation comprising the empty viral capsids and the full viral capsids, wherein the viral capsid preparation comprises a first salt concentration; (ii) contacting a volume of the viral capsid preparation with a stationary phase surface in an anion exchange system and passing the volume of viral capsid preparation through the anion exchange system to generate a flowthrough comprising empty viral capsids; and (iii) conducting an one-step elution step by contacting the stationary phase surface with an elution solution comprising a second salt concentration and passing the elution solution through the anion exchange system to generate an eluent comprising full viral capsids.

The method may comprise: (i) providing a viral capsid preparation comprising the empty viral capsids and the full viral capsids, wherein the viral capsid preparation comprises a first salt concentration; (ii) contacting a volume of the viral capsid preparation with a stationary phase surface in an anion exchange system and passing the volume of viral capsid preparation through the anion exchange system to generate a flowthrough comprising empty viral capsids; and (iii) conducting the one-step isocratic elution step by contacting the stationary phase surface with an elution solution comprising a second salt concentration and passing the elution solution through the anion exchange system to generate an eluent comprising full viral capsids.

The methods disclosed herein do not require repeating the contacting step (ii) and/or the elution step (iii). In some preferred embodiments in which the contacting step (ii) and elution step (iii) are repeated, the salt concentration of the elution solution between elution step remains unchanged, meaning the second salt concentration of the elution solution remains constant within the elution series. In some preferred embodiments in which the contacting step (ii) and elution step (iii) are repeated, the pH value and salt concentration of the elution solution between elution step remains unchanged, meaning the pH value and the second salt concentration of the elution solution remains constant within the elution series.

In preferred embodiments, the methods disclosed herein comprise one contacting step (ii). In preferred embodiments, the methods disclosed herein comprise one one-step elution (iii). In preferred embodiments, the methods disclosed herein comprise one contacting step (ii) and one one-step elution (iii). To achieve the full and empty capsid separation, the method disclosed herein does not require repeating the method steps (i)-(iii) disclosed herein. In this context it is understood that the elute comprising the eluted full capsids comprises the second salt concentration which differs from the first salt concentration of the viral capsid preparation, rendering the elute unsuitable to be used in the contacting step (ii). The method disclosed herein further does not require conditioning the elute e.g., by dilution to reduce the salt concentration. The rapid and efficient separation of the novel method disclosed herein can be achieved by using a single contacting step (ii) and a single elution step (iii). In preferred embodiments, a single stationary phase (anion exchange medium) is used.

In some embodiments, the viral capsid preparation comprises less than 5 × 10¹³ viral particles per ml (vp/ml), less than 5 × 10¹⁴, less than 4 × 10¹⁴ vp/ml, less than 3 × 10¹⁴ vp/ml, less than 2 × 10¹⁴ vp/ml, or less than 1 × 10¹⁴ vp/ml. For example, the viral capsid preparation can comprise less than 5 × 10¹⁵ vp/ml, less than 4 × 10¹⁵ vp/ml, less than 3 × 10¹⁵ vp/ml, less than 2 × 10¹⁵ vp/ml, less than 1 × 10¹⁵ vp/ml, less than 5 × 10¹⁶ vp/ml, less than 4 × 10¹⁶ vp/ml, less than 3 × 10¹⁶ vp/ml, less than 2 × 10¹⁶ vp/ml, or less than 1 × 10¹⁶ vp/ml.

In some embodiments, the method comprises an optional equilibrating step, such as an equilibrating step before contacting the viral capsid preparation with the equilibrated stationary phase surface in the contacting step (ii). In some embodiments, the method comprises one or more equilibrating steps. For example, the stationary phase surface can be equilibrated using an equilibrating solution. Any of a variety of equilibrating solutions may be suitable for use in accordance with methods disclosed herein. In some embodiments, the pH and salt concentration of the equilibrating solution is chosen based on characteristics of the viral capsid preparation and/or the type of stationary phase being used. In some embodiments, the equilibrating solution comprises the pH value, components, and component concentrations (such as salts and buffering components) of the viral capsid preparation.

In some embodiments, the method comprises an optional washing step. In some embodiments, the method comprises one or more wash steps. For example, the viral capsid preparation may be contacted with the stationary phase surface in step (ii), followed by a washing step of the stationary phase surface with one or more washing solutions to separate (and optionally collect separated fractions) empty and full capsids from one another. In some embodiments, the method does not comprise a washing step. In some embodiments, the washing solution comprises the pH value, components, and component concentrations (such as salts and buffering components) of the viral capsid preparation.

In some embodiments, the method is used for analytic sample preparation, meaning the method is not used to prepare AAV samples for gene therapy. In some such embodiments, the washing solution can comprise a quaternary ammonium salt. In some embodiments, the quaternary ammonium salt is a tetraalkylammonium salt, e.g., a tetraalkylammonium chloride or a tetraalk l ammonium acetate. In some embodiments, the quaternary ammonium salt is a tetraalkydammonium chloride selected from the group consisting of tetramethylammonium chloride (TMAC), tetraethylammonium chloride (TEAC), tetrapropylammonium chloride (TPAC), tetrabutylammonium chloride (TBAC), benzyltributylammonium chloride (BTBAC), or any combination(s) thereof. In some embodiments, the quaternary' ammonium salt is tetraethylammonium chloride (TEAC). In some embodiments, the washing solution comprise a quaternary ammonium salt less than 200 mM, less than 180 mM, less than 160 mM, less than 150 mM, less than 140 mM, less than 130 mM, less than 120 mM, less than 100 mM, less than 90 mM, less than 80mM, less than 70 mM, less than 60 mM, less than 50 mM, less than 40 mM, less than 30 mM, less than 20 mM, less than 10 mM, less than 5 mM, or less than 1 mM of a quaternary ammonium salt.

In some embodiments, the washing solution does not comprise a quaternary ammonium salt.

The method disclosed herein may comprise:
(i) providing the viral capsid preparation comprising the empty viral capsids and the full viral capsids, wherein the viral capsid preparation comprises the first salt concentration;
(ii) optionally equilibrating the stationary phase surface;
   contacting the viral capsid preparation with the equilibrated stationary phase surface allowing binding of the full viral capsids to the stationary phase surface, wherein the empty viral capsids at least partially do not bind to the stationary phase surface;
   optionally washing the stationary phase surface; and
(iii) conducting a one-step and/or gradient elution in which the full capsids are eluted by contacting the stationary phase obtained in step (ii) with an elution solution comprising a second salt concentration.

When using the one-step elution in step (iii), it is understood that a series of two or more separate solutions are not used during the elution, wherein each of which has different pH values and/or different fixed concentrations of one or more salts relative to another solution in the series.

In some preferred embodiments, the elution is a one-step elution (e.g., comprising a constant salt concentration and a constant pH value). In some particularly preferred embodiments, the one-step elution is an isocratic elution (i.e., one-step isocratic elution). As used herein, the term "isocratic elution" refers to an elution wherein the pH and the concentration of all salts in the elution solution is kept constant during the elution step. Preferably, when using the one-step elution (such as a one-step isocratic elution) in step (iii), a single elution solution is used. As referred herein, a single elution solution refers to a solution comprising a specific (fixed) pH value and specific (fixed) salt concentrations, even if provided in separate containers.

It is understood that the method disclosed herein comprises a one-step elution, i.e., preferably not comprise an "isocratic elution gradient", meaning a gradient wherein the composition of the mobile phase is changed in two or more steps during a single chromatographic run. In each step of an isocratic elution gradient, the mobile phase is kept at the same composition (e g., constant concentration) until a subsequent step in the chromatographic run, at which time the composition of the mobile phase is changed such that the concentration of a component of the mobile phase is increased relative to the concentration of the component in a previous step. Thus, for example, an isocratic elution gradient of MgCl₂ involves using various steps, with a constant MgCl₂ concentration at each individual step, but with increasing MgCl₂ concentrations from one step to a subsequent step.

The method disclosed herein does not comprise a stepped gradient elution, preferably not a stepped salt gradient elution and/or stepped pH gradient elution, more preferably not a stepped salt and pH gradient elution. A stepped gradient elution as used herein refers to an elution comprising at least two steps, wherein the elution solution used in each step differs from the other by its salt concentration and/or pH value.

In some embodiments, the pH of the viral capsid preparation solution and/or the pH of the elution solution are within a range of about 6 to about 10, preferably about 7 to about 9, preferably about 8 to about 9. In the pH can be in the range of 6.0 ± 0.2 to 10.0 ± 0.2, 6.5 ± 0.2 to 9.5 ± 0.2, 7.0 ± 0.2 to 9.0 ± 0.2, 7.5 ± 0.2 to 8.50 ± 0.2, or 8.0 ± 0.2 to 8.5 ± 0.2. It is understood that the exact pH may vary among (AAV) serotypes and potentially among different recombinant constructs within a serotype. The method disclosed herein does not require a pH gradient or pH step gradient to elute bound full capsids. Further, no shift in pH of the viral capsid preparation and elution solution is required, allowing for mild polishing conditions in order to conserve full capsid stability and potency.

The pH of the viral capsid preparation can be within a range of about 6 to about 9, about 6 to about 8, about 6 to about 7, or within a range of about 7 to about 10, about 8 to about 10, or about 9 to about 10. For example, the pH of the viral capsid preparation solution can be about 6, about 6.5, about 7, about 7.5, about 8, about 8.5, about 9, about 9.5, or about 10. the pH of the elution solution can be about 6, about 6.5, about 7, about 7.5, about 8, about 8.5, about 9, about 9.5, or about 10. The pH of the elution solution can be within a range of about 6 to about 9, about 6 to about 8, about 6 to about 7, or within a range of about 7 to about 10, about 8 to about 10, or about 9 to about 10. For example, the pH of the elution solution can be about 6, about 6.5, about 7, about 7.5, about 8, about 8.5, about 9, about 9.5, or about 10. Preferably, the pH of the viral capsid preparation solution and the pH of the elution solution differ about 1 pH unit or less, about 0.9 pH units or less, about 0.8 pH units or less, preferably about 0.7 pH units or less, preferably about 0.6 pH units or less, preferably about 0.5 pH units or less, preferably about 0.4 pH units or less, preferably about 0.3 pH units or less, more preferably about 0.2 pH units or less, most preferably about 0.1 pH units or less. In some embodiments, the pH of the viral capsid preparation and the pH of the elution solution do not substantially differ. In some embodiments, the method can be conducted using a constant pH during the contacting step (ii) and elution step (iii).

In some embodiments, the viral capsid preparation is an aqueous preparation, preferably a buffered aqueous preparation. In some embodiments, the elution solution is an aqueous solution, preferably a buffered aqueous solution.

In some preferred embodiments, the viral capsid preparation solution and/or elution solution comprises a buffering component maintaining the pH at about 6 to about 10, preferably about 7 to about 9, preferably about 8 to about 9. For example, the buffering component maintaining the pH at about 6 to about 9, about 6 to about 8, about 6 to about 7, or within a range of about 7 to about 10, about 8 to about 10, or about 9 to about 10. Any suitable buffering component known to the skilled person can be used in context of the present disclosure. The pH is preferably measured at 20 °C.

Non-limiting examples of suitable buffer systems include ADA, PIPES, ACES, MOPSO, BES, MOPS, TES, HEPES, DPSO, Tricine, Glycylglycine (Gly-Gly), EPPS, BICINE, TAPS, AMPD, AMPSO, CHES, Tris, or bis-Tris Propane-based buffers, such as a 20 mM bis-Tris Propane system. An exemplary buffer component comprises or is Tris, wherein the viral capsid preparation solution and/or elution solution can comprise about 5 mM to 200 mM Tris, preferably about 10 to about 100 mM Tris, such as 20 mM or 25 mM Tris.

In some embodiments, the salt is a chaotropic salt. In some embodiments, the viral capsid preparation comprises a first chaotropic salt concentration and the elution solution comprises a second chaotropic salt concentration. It is understood that the viral capsid preparation and/or elution solution may comprise different salts (one or more salts), wherein at least one salt is a chaotropic salt. In some embodiments, the salt (e.g., chaotropic salt) comprises a monovalent or divalent cation. In some embodiments, the salt (e.g., chaotropic salt) comprises a monovalent cation. In some embodiments, the salt (e.g., chaotropic salt) comprises a divalent cation.

In some embodiments, the salt (e.g., chaotropic salt) comprises Mg²⁺, Ca⁺, or Na⁺ or any combination thereof. In some embodiments, the salt (e.g., chaotropic salt) comprises Mg²⁺. In some embodiments, the salt (e.g., chaotropic salt) comprises Ca⁺. In some embodiments, the salt (e.g., chaotropic salt) comprises Na⁺. In some embodiments, the salt (e.g., chaotropic salt) comprises acetate, chloride, or sulfate or any combination thereof. In some embodiments, the salt (e.g., chaotropic salt) comprises acetate. In some embodiments, the salt (e.g., chaotropic salt) comprises chloride. In some embodiments, the salt comprises sulfate.

In some embodiments, the salt comprises or is selected from magnesium sulfate, sodium sulfate, calcium sulfate, magnesium chloride, sodium chloride, calcium chloride, magnesium acetate, sodium acetate, calcium acetate, or any combination thereof. In some embodiments, the salt (e.g., chaotropic salt) comprises or is selected from magnesium sulfate, sodium sulfate, calcium sulfate, magnesium chloride, calcium chloride, magnesium acetate, sodium acetate, calcium acetate, or any combination thereof. In some preferred embodiments, the salt (e.g., chaotropic salt) comprises or is magnesium chloride (MgCl₂).

In some embodiments, the viral capsid preparation and/or elution solution comprises 20 mM NaCl or less, preferably 15 mM NaCl or less, 10 mM NaCl or less, 9 mM NaCl or less, 8 mM NaCl or less, 7 mM NaCl or less, 6 mM NaCl or less 5 mM NaCl or less, 4 mM NaCl or less, 3 mM NaCl or less, 2 mM NaCl or less, 1 mM NaCl or less, 0.5 mM NaCl or less, 0.4 mM NaCl or less, 0.3 mM NaCl or less, 0.2 mM NaCl or less, or 0.1 mM NaCl or less.

In some embodiments, the salt (e.g., chaotropic salt) comprises or is selected from magnesium sulfate, sodium sulfate, or calcium sulfate or any combination thereof. In some embodiments, the salt comprises or is selected from magnesium chloride, sodium chloride, or calcium chloride or any combination thereof. In some embodiments, the salt (e.g., chaotropic salt) comprises or is selected from magnesium acetate, sodium acetate, or calcium acetate or any combination thereof. It is understood by the skilled person that sodium chloride is not a chaotropic salt.

In some embodiments, the viral capsid preparation comprises a first magnesium chloride concentration. In some embodiments, the first salt concentration is a first magnesium chloride concentration. In some embodiments, the elution solution comprises a second magnesium chloride concentration. In some embodiments, the second salt concentration is a second magnesium chloride concentration.

In some embodiments, the full viral capsids and empty viral capsids are *Parvoviridae* capsids. In some embodiments, the full viral capsids and empty viral capsids are *Dependoparvovirus* capsids. In some embodiments, the full viral capsids and empty viral capsids are *Adeno-associated viruses* (AAV) capsids.

In some embodiments, the AAV has a serotype selected from the group consisting of AAV1, AAV2, AAV3, AAV4, AAV5, AAV6, AAV7, AAV8, AAV9, AAV10, AAV11, AAV12, AAV13, a synthetic recombinant serotype, or a recombinant hybrid serotype, or combinations thereof.

The full and empty viral capsids in any given sample may belong to any serotype, including natural and recombinant serotypes. It will be understood that capsids of different serotypes have different charge properties, including different levels of charge distinction between empty and full capsids, and that such differences may also be apparent among different recombinant constructs within a serotype. Thus, some degree of optimization may be required when applying the method disclosed herein to specific AAV serotypes.

In some embodiments, flow-through fluid comprising unbound empty capsids is obtained in step (ii).

In some embodiments, a UV spectrophotometry method is used to assess a viral capsid preparation, a fraction (e.g., flow-through fluid), or the elute comprising full capsids. For example, the amount of light at wavelengths at or around 254 nm and/or 260 nm that is absorbed by a sample is generally proportional to the concentration of nucleic acids in the sample. Additionally, proteins have a greater absorbance at 280 nm (A280) than they do at 254 nm (A254) or 260 nm (A260); the inverse is true for nucleic acids, which have a greater A254 or A260 than A280. Thus, full capsids, which have a greater amount of DNA than empty capsid particles, will have a greater A254/A280 or A260/A280 ratio than empty capsid particles. This difference can be exploited to assess the relative amounts of full and empty capsids in a sample. In some embodiments, one or more of A254, A260, A280, A254/A280 ratio, or A260/A280 ratio, is assessed. In some embodiments of the methods of the disclosure, the analyzing the empty and full capsid content comprises determining the A254/A280 ratio of the collected portion of the flowthrough using UV spectrophotometry. In some embodiments, the A254/A280 ratio is directly proportional to the presence of full capsids in a sample, e.g., the flowthrough.

In some embodiments, the presence and/or amount(s) of full and/or empty capsids in the viral capsid preparation and/or one or more eluents (or elution fractions) is assessed. Various methods are known in the art to determine or quantify the presence of full or empty capsids; many of these methods can also be used to determine amounts, e g., relative amounts of full and empty capsids. Examples of such methods include, but are not limited to, transmission electron microscopy (TEM), sedimentation velocity-analytical ultracentrifugation (SV-AUC), charge detection mass spectrometry (CDMS), anion exchange high performance liquid chromatography (AEX-HPLC), UV spectrophotometry, and measuring capsid and genome copies by ELISA and qPCR, e.g., for quality control purposes.

In some embodiments, the majority of empty capsids do not bind to the stationary phase surface. The flowthrough obtained by step (ii) can comprise at least about 50% (e.g., at least about 50%, at least about 55%, at least about 60%, at least about 65%, at least about 70%, at least about 75%, at least about 80%, at least about 85%, at least about 90%, at least about 95% or about 100%) of the empty capsids present in the volume of the viral capsid preparation.

In some embodiments, the first salt concentration is in the range of about 0.01 to about 100 mM, such as from about 0.01 to about 95 mM, about 0.01 to about 90 mM, about 0.01 to about 85 mM, about 0.01 to about 80 mM, about 0.01 to about 75 mM, about 0.01 to about 70 mM, about 0.01 to about 65 mM, about 0.01 to about 60 mM, about 0.01 to about 55 mM, about 0.01 to about 50 mM, about 0.01 to about 45 mM, about 0.01 to about 40 mM, about 0.01 to about 35 mM, about 0.01 to about 30 mM, about 0.01 to about 25 mM, about 0.02 to about 20 mM, about 0.03 to about 15 mM, about 0.04 to about 10 mM, or about 0.05 to about 5 mM, and all integers including and in between about 0.1 mM to about 100 mM.

In some embodiments, the second salt concentration is in the range of about 0.1 to about 140 mM, such as from about 0.1 to about 135 mM, about 0.1 to about 130 mM, about 0.1 to about 125 mM, about 0.1 to about 120 mM, about 0.1 to about 115 mM, about 0.1 to about 110 mM, about 0.1 to about 100 mM, about 0.1 to about 95 mM, about 0.1 to about 90 mM, about 0.1 to about 85 mM, about 0.2 to about 80 mM, about 0.3 to about 75 mM, about 0.4 to about 70 mM, about 0.5 to about 65 mM, about 0.6 to about 60 mM, about 0.7 to about 55 mM, about 0.9 to about 50 mM, about 0.9 to about 45 mM, about 1.0 to about 40 mM, about 1.2 to about 35 mM, about 1.3 to about 30 mM, about 1.4 to about 35 mM, about 1.5 to about 30 mM, about 1.6 to about 25 mM, about 1.7 to about 20 mM, about 1.8 to about 15 mM, about 1.9 to about 10 mM, about 2 to about 5 mM, and all integers including and in between about 0.1 mM to about 140 mM.

It is understood that the exact salt concentration may vary among AAV serotypes and potentially among different recombinant constructs within a serotype.

In some embodiments, the full viral capsids and empty viral capsids are AAV2 capsids. In some such embodiments, the first salt concentration is in the range of about 0.01 to about 10 mM, about 0.02 to about 8 mM, about 0.03 to about 7 mM, about 0.04 to about 6 mM, about 0.05 to about 5 mM, about 0.06 to about 4 mM, about 0.07 to about 3 mM, or about 0.08 to about 2 mM.

In some such embodiments, the second salt concentration is in the range of about 0.1 to about 15 mM, about 0.5 to about 13 mM, about 1 to about 11 mM, about 1.5 to about 9 mM, about 2 to about 7 mM, about 2.5 to about 5 mM, or about 3 to about 4 mM.

In some embodiments, the full viral capsids and empty viral capsids are AAV5 capsids. In some such embodiments, the first salt concentration is in the range of about 0.5 to about 100 mM, about 1 to about 80 mM, about 2 to about 60 mM, about 3 to about 40 mM, about 4 to about 30 mM, about 5 to about 25 mM, about 6 to about 20 mM, or about 7 to about 15 mM. In some such embodiments, the second salt concentration is in the range of about 1 to about 140 mM, about 3 to about 120 mM, about 4 to about 100 mM, about 5 to about 80 mM, about 8 to about 60 mM, about 9 to about 40 mM, or about 10 to about 30 mM.

In some embodiments, the full viral capsids and empty viral capsids are AAV8 capsids. In some such embodiments, the first salt concentration is in the range of about 0.1 to about 100 mM, about 0.5 to about 80 mM, about 1 to about 60 mM, about 1.5 to about 40 mM, about 2 to about 30 mM, about 2.5 to about 20 mM, or about 3 to about 10 mM. In some such embodiments, the second salt concentration is in the range of about 1 to about 140 mM, about 2 to about 120 mM, about 3 to about 100 mM, about 4 to about 80 mM, about 5 to about 60 mM, about 6 to about 40 mM, or about 7 to about 20 mM.

Exemplary salt concentration of the viral capsid preparation and elution solution suitable for use in the method disclosed herein using the exemplary stationary phase systems PrimaS and Sartobind Q are provided in table 1.

**Table 1: Exemplary salt concentrations used to separate full and empty AAV2, AAV5, and AAV8 capsids according to the method disclosed herein.**

| | **PrimaS** | | | **Sartobind Q** | | |
|---|---|---|---|---|---|---|
| **Serotype** | **AAV8** | **AAV5** | **AAV2** | **AAV8** | **AAV5** | **AAV2** |
| **MgCl2 [mM]** | FT: 3.6 | FT: 11.6 | FT: 0.1 | FT: 24.6 | FT: 28.6 | FT: 15.4 |
| | Elu.: 9.2 | Elu.: 1.2 | Elu.: 3 | Elu.: 30.8 | Elu.: 32.7 | Elu.: 20.2 |

| | | | | | | |
|---|---|---|---|---|---|---|
| FT (Flowthrough) = MgCl₂ concentration [in mM] of the viral capsid preparation; Elu. (Elution) = MgCl₂ concentration [in mM] of the elution solution. | | | | | | |

In some embodiments, the viral capsid preparation is a crude cell harvest or a crude cell lysate. In some embodiments, the viral capsid preparation is a purified crude cell harvest or a purified crude cell lysate. In some embodiments, the viral capsid preparation is a purified crude cell harvest. It is understood that not all serotypes require a lysis step since e.g., the viral capsids may not remain intracellular. In some embodiments, the viral capsid preparation is a purified crude cell lysate. The viral capsid preparation may be in the form of a cell culture harvest, a cell lysate, a partially purified preparation such an elution fraction from an affinity chromatography column, a partially purified fraction eluted from an ion exchange chromatography column, a partially purified fraction eluted from a hydrophobic interaction chromatography column, or capsid mixtures from any other purification method or combination of methods employed to process the viral capsid.

In some embodiments, the method further comprises the step of providing a crude cell harvest or crude cell lysate and purifying the crude cell harvest or crude cell lysate to obtain the viral capsid preparation of step (i) (e.g., by steric exclusion chromatography). In preferred embodiments, the purified crude cell harvest or purified crude cell lysate comprises the first salt concentration. In preferred embodiments, the purified crude cell harvest or purified crude cell lysate comprises the desired pH, salt composition, and first salt concentration. The method disclosed herein may not require adapting the pH and/or salt composition and/or salt concentration of the crude cell harvest or crude cell lysate. The method disclosed herein may not require adapting the pH and/or salt composition and/or salt concentration of the purified crude cell harvest or purified crude cell lysate. For example, the crude cell harvest or crude cell lysate (e.g., purified crude cell harvest or purified crude cell lysate) may not need to be conditioned by titration of pH, dilution to reduce the salt concentration, and/or buffer exchange techniques.

In some embodiments, the cells are HEK293 cells, HELA cells, CHO cells, insect cells, or derivatives thereof.

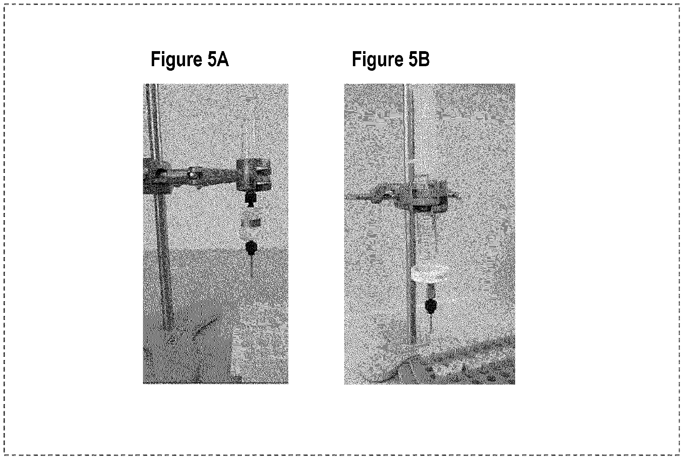

In some embodiments, the method is conducted in a syringe-based system. The setup of an exemplary syringe-based systems is shown in figure 5A (CIMmultus PrimaS^{®}) and figure 5B (Sartobind Q^{®}). Exemplary syringe-based system suitable for the method disclosed herein are CIMmultus PrimaS^{®} and Sartobind Q^{®} Membrane Adsorbers (both available from Sartorius). While the PrimaS column is characterized by a monolithic structure with a multimodal ligand that combines hydrogen bonding with a weak anion exchanger, the Sartobind Q is characterized by a quaternary amine ligand and thus a strong anion exchanger. The method disclosed herein is however not limited to particular syringe-based systems. Some degree of optimization (e.g., of the pH value and/or salt concentration of the viral capsid preparation and/or elution solution) may however be required when applying the method disclosed herein to different stationary phase systems and syringe-based systems.

In some embodiments, the stationary phase is a chromatographic stationary phase. In some embodiments, the stationary phase is characterized by a monolithic structure, membrane, or resin with a multimodal ligand that combines hydrogen bonding with a weak anion exchanger. In some embodiments, the stationary phase comprises a quaternary amine ligand. In some embodiments, the stationary phase surface comprises a quaternary amine ligand (as affinity ligand).

In some embodiments, the stationary phase surface is not modified with dextran. In some embodiments, the stationary phase surface does not comprise dextran surface extenders. Meaning dextran is not utilized as a surface extension of the base matrix for expanding the three dimensional spacing between ligands relative to a stationary phase surface with the same ligand density.

It is understood that the viral capsid preparation and/or elution solution may comprise further components for the purpose of stabilizing full capsids, for improving capsid solubility, and/or suppressing non-specific interactions between capsids and the stationary phase surface that might reduce separation efficiency or reduce full capsid recovery.

The invention further provides a kit comprising:
(i) a first container comprising a preparation solution with a first salt concentration; and
(ii) a second container comprising an elution solution with a second salt concentration,
wherein the first salt concentration is lower than the second salt concentration.

In some embodiments, the first container comprises up to 500 ml, up to 400 ml, up to 350 ml, up to 300 ml, up to 250 ml, or up to 200 ml of the preparation solution. In some embodiments, the first container comprises at least 20 ml, at least 30 ml, at least 40 ml, or at least 50 ml of the preparation solution. In some embodiments, the first container comprises 20 to 500 ml, 30 to 400 ml, 40 to 300 ml, or 50 to 200 ml of the preparation solution.

In some embodiments, the second container comprises up to 500 ml, up to 400 ml, up to 350 ml, up to 300 ml, up to 250 ml, up to 200 ml of the elution solution. In some embodiments, the second container comprises at least 20 ml, at least 30 ml, at least 40 ml, or at least 50 ml of the elution solution. In some embodiments, the second container comprises 20 to 500 ml, 30 to 400 ml, 40 to 300 ml, or 50 to 200 ml of the elution solution.

In some embodiments, the kit comprises a stationary phase surface allowing binding of the full viral capsids to the stationary phase surface.

In some embodiments, the kit comprises a syringe-based system comprising the stationary phase.

It is understood that the preparation solution and elution solution can comprise the pH, the components (e.g., salt and buffering components), and component concentration of the viral capsid preparation and elution solution described for the method disclosed herein.

In some embodiments, the pH of the viral capsid preparation and the pH of the elution solution do not substantially differ. In some embodiments, the pH of the preparation solution and the pH of the elution solution are within a range of about 6 to about 10, preferably about 7 to about 9, preferably about 8 to about 9. For example, the pH of the preparation solution can be about 6, about 6.5, about 7, about 7.5, about 8, about 8.5, about 9, about 9.5, or about 10. the pH of the elution solution can be about 6, about 6.5, about 7, about 7.5, about 8, about 8.5, about 9, about 9.5, or about 10. The pH of the preparation elution and the pH of the elution solution can be within a range of about 6 to about 9, about 6 to about 8, about 6 to about 7, or within a range of about 7 to about 10, about 8 to about 10, or about 9 to about 10. Preferably, the pH of the preparation solution and the pH of the elution solution differ about 1 pH unit or less, about 0.9 pH units or less, about 0.8 pH units or less, preferably about 0.7 pH units or less, preferably about 0.6 pH units or less, preferably about 0.5 pH units or less, preferably about 0.4 pH units or less, preferably about 0.3 pH units or less, more preferably about 0.2 pH units or less, most preferably about 0.1 pH units or less.

In some preferred embodiments, the preparation solution and/or elution solution comprises a buffering component maintaining pH at about 6 to about 10, preferably about 7 to about 9, preferably about 8 to about 9. For example, the buffering component maintaining the pH at about 6 to about 9, about 6 to about 8, about 6 to about 7, or within a range of about 7 to about 10, about 8 to about 10, or about 9 to about 10.

In some embodiments, the salt comprises or is selected from magnesium sulfate, sodium sulfate, calcium sulfate, magnesium chloride, sodium chloride, calcium chloride, magnesium acetate, sodium acetate, calcium acetate, or any combination thereof, preferably wherein the salt comprises or is selected from magnesium sulfate, sodium sulfate, calcium sulfate, magnesium chloride, calcium chloride, magnesium acetate, sodium acetate, calcium acetate, or any combination thereof. In some embodiments, the salt comprises or is selected from magnesium sulfate, sodium sulfate, or calcium sulfate or any combination thereof.

In some embodiments, the salt comprises or is selected from magnesium chloride, sodium chloride, or calcium chloride or any combination thereof. In some embodiments, the viral capsid preparation and/or elution solution comprises 20 mM NaCl or less, preferably 15 mM NaCl or less, 10 mM NaCl or less, 9 mM NaCl or less, 8 mM NaCl or less, 7 mM NaCl or less, 6 mM NaCl or less 5 mM NaCl or less, 4 mM NaCl or less, 3 mM NaCl or less, 2 mM NaCl or less, 1 mM NaCl or less, 0.5 mM NaCl or less, 0.4 mM NaCl or less, 0.3 mM NaCl or less, 0.2 mM NaCl or less, or 0.1 mM NaCl or less.

In some embodiments, the salt comprises or is selected from magnesium acetate, sodium acetate, or calcium acetate or any combination thereof.

The preparation solution disclosed herein preferably comprises the pH value, components, and component concentrations of the viral capsid preparation disclosed herein.

The elution solution disclosed herein preferably comprises the pH value, components, and component concentrations of the elution solution disclosed herein.

The invention further provides the use of the kit disclosed herein in the method disclosed herein.

The invention is further explained by the following non-limiting examples.

### EXAMPLES

Examples 1-4 were performed using the materials and methods set out below.

### AAV production and harvest

AAV2, AAV5, and AAV8 were produced via transient transfection of HEK293 cells (Expi293F Inducible Cells, Thermo Fisher Scientific, Waltham, MA) in both non-baffled glass shake flasks and a 10 L glass Univessel^{®} bioreactor (Sartorius, Göttingen, Germany). Both systems used FreeStyle 293 Expression Medium (Thermo Fisher Scientific) as the growth medium. In the shake flask system, cells were cultivated at 130 rpm, 8% CO2, and 80% humidity in an orbital shaker (Kuhner, Birsfelden, Switzerland). Within the 10 L bioreactor, the cultivation conditions were regulated by the BIOSTAT^{®} DCU controller unit (Sartorius) to maintain set points of 201 rpm, 40% dissolved oxygen, and pH 7.25.

Furthermore, to prevent foaming within the bioreactor, the cultivation medium within the bioreactor was supplemented with antifoam C (Sigma-Aldrich, Darmstadt, Germany) at a final concentration of 0.0002% (v/v). AAV production processes were inoculated at 3 × 10⁵ viable cells/mL. After reaching a cell density of 1.3 × 10⁶ viable cells/mL, transfection was performed using a dual plasmid system, with 0.55 µg/mL DNA per 10⁶ viable cells and FectoVIR-AAV (Polyplus, Illkirch, France) as the transfection reagent in a 1:1 ratio. The plasmids pAAV-ssGFP (vector plasmid comprising GFP as transgene) and pDP2/pDP5/pDP8.ape (packaging and helper plasmid for each respective serotype) were used for transfection in a molar ratio of 4.5:1.

Plasmids were sourced from PlasmidFactory (Bielefeld, Germany).

At 72 h post-transfection, AAV vectors were harvested. To initiate cell lysis, the cell broth was treated with a 20x lysis buffer (1 M Tris pH 8.0 at 20°C, 40 mM MgCl₂ (both Carl Roth, Karlsruhe, Germany), 20% (v/v) Tween 20 (Sigma-Aldrich)) and Denarase (c-Lecta, Leipzig, Germany) at a final concentration of 50 U/mL. The mixture was then incubated at 37°C for 1.5 h. Subsequently, the lysed cultures were treated with 500 mM NaCl and incubated at 37°C for another 30 min.

### Purification of AAV from crude cell lysates

Purification of AAV crude cell lysates was performed by alluvial filtration as previously reported in Meierrieks, F., Pickl, A., and Wolff, M.W. (2023). A robust and efficient alluvial filtration method for the clarification of adeno-associated viruses from crude cell lysates. Journal of biotechnology 117, 3199.

Briefly, the crude cell lysate was mixed with 11.25 g/L of Sartoclear Dynamics^{®} Lab Filter Aid (Sartorius) and clarified using Sartolab^{®} RF1000 filters (0.22 µm; PES; Sartorius). The clarified AAV sample was kept on ice and further purified by affinity chromatography using AAVX pre-packed columns (1 mL, Thermo Fisher Scientific) and an ÄKTATM avant 25 (Cytiva, Marlborough, MA, USA) chromatography system.

Four distinct buffers were prepared as follows:
(A) 25 mM Tris pH 8.5, 5 mM MgCl₂, 250 mM NaCl,
(B1) 20 mM Glycine pH 2.3, 2 mM MgCl₂, 200 mM NaCl (for AAV8),
(B2) 20 mM Glycine pH 1.7, 2 mM MgCl₂, 200 mM NaCl (for AAV2 and AAV5), and
(C) 1 M Tris pH 8.5.

There four buffers are referred to as binding (A), elution (B), and neutralization buffer (C).

All chemicals were purchased from Carl Roth. The column was first equilibrated with 20 column volumes (CV) of binding buffer. Subsequently, a total of 750-1000 mL of clarified AAV sample was loaded per run in a downflow direction onto the column at a flow rate of 0.5-0.7 mL/min. Following sample application, the column was washed with 10 column volumes (CV) of binding buffer. Before elution, 50 µL of neutralization buffer was added to each well of the fraction collector plate. The AAV bound to the column were then eluted with elution buffer in an upflow direction in 0.45 mL fractions. Peak fractions were pooled, aliquoted, and stored at -80°C until further use.

### Anion exchange separation of full from empty AAV capsids

For the separation of full from empty AAV capsids, two different AEC devices were applied. Experiments were performed using the multimodal monolithic column CIMmultus/CIMmic PrimaS (2 µm pore size, 1 mL, and 0.1 mL, Sartorius BIA Separations, Ajdovscina, Slovenia) that combines hydrogen bonding with weak AEC. The second device used was the strong AEC membrane adsorber (MA) Sartobind Lab Q (0.41 mL, Sartorius).

Two different buffers were prepared as follows: (A) 50 mM Tris pH 8.5, 2 mM MgCl₂, and (B) 50 mM Tris pH 8.5, 50 mM MgCl₂, hereafter referred to as MgCh loading and elution buffer. Only the loading buffer for the AAV2 experiments on the PrimaS column did not contain MgCl₂.

Stationary phases were cleaned and regenerated based on manufacturer's recommendations. Experiments were conducted on an ÄKTATM avant 25 (Cytiva) chromatography system at a flow rate of 2 mL/min. All chemicals were purchased from Carl Roth.

Sartobind Q: Linear and step gradient elution experiments for MgCl₂ were conducted with a total of 8 × 10¹²-2 × 10¹³ capsids of affinity-purified AAV8, AAV5, or AAV2. The AAV material was diluted 1:20-80 in the respective loading buffer and loaded onto the Sartobind Q via a 5 mL or 10 mL sample loop. After washing with another 3-6 mL of loading buffer, linear gradient elution was performed from 0-100% of MgCl₂ elution buffer over 4 min. Step gradient elution experiments employed 5% increments of MgCl₂ elution buffer. For the flow-through polishing approach, 8 × 10¹²-3.9 × 10¹³ capsids of affinity-purified AAV8, AAV5, or AAV2 were diluted 1:50-80 and MgCl₂ concentrations were adjusted to 24.6 mM for AAV8, 28.6 mM for AAV5, and 15.4 mM for AAV2 by appropriate mixing of the MgCl₂ loading and elution buffer. Sample application was performed using a 10 mL sample loop, followed by a washing step for 5-8 mL with a loading and elution buffer mixture containing the same MgCl₂ concentration as during sample application. Isocratic elution was then achieved by increasing the MgCl₂ concentration to 30.8 mM for AAV8, 32.7 mM for AAV5, and 20.2 mM for AAV2.

CIMmultus PrimaS: Linear and step gradient elution experiments for MgCl₂ were executed using 8 × 10¹²- 2.9 × 10¹³ capsids of affinity-purified AAV8, AAV5, or AAV2. The AAV preparations were diluted 1: 10-20 in the respective loading buffer and loaded onto the PrimaS column using a 5 mL sample loop. A subsequent wash step with another 3-8 mL of loading buffer preceded a linear gradient elution ranging from 0 to 30-50% of gradient elution experiments for MgCl₂ elution buffer over 3 min. For step gradient elution, 2-3% increments of gradient elution experiments for MgCl₂ elution buffer were utilized. For the gradient elution experiments for MgCl₂ flow-through polishing method, 8 × 10¹²-2.9 × 10¹³ capsids of affinity-purified AAV8, AAV5, or AAV2 were diluted 1:10-16 and MgCl₂ concentrations were adjusted to 3.6 mM for AAV8, 11.6 mM for AAV5, and 0.1 mM for AAV2 by appropriate mixing of the gradient elution experiments for MgCl₂ loading and elution buffer. Sample application was performed using a 5 mL sample loop, followed by a washing step for 3-4 mL with a loading and elution buffer mixture containing the same gradient elution experiments for MgCl₂ concentration as during sample application. Isocratic elution was accomplished by increasing the gradient elution experiments for MgCl₂ concentration to 9.2 mM for AAV8, 21.2 mM for AAV5, and 3 mM for AAV2.

Syringe approach: For AAV8 flow-through polishing, an FPLC-independent protocol with manual syringe operation was implemented using the Sartobind^{®} Lab Q (0.41 mL, Sartorius) and CIMmic PrimaS (2 µm pore size, 0.1 mL, Sartorius BIA Separations). All steps were performed at a flow rate of around one drop per second. A total of 2.3 × 10¹³-3.6 × 10¹³ capsids of affinity-purified AAV8 were diluted 1:50 or 1:15 and adjusted to 24.6 mM or 3.6 mM gradient elution experiments for MgCl₂ for the Sartobind Q and the PrimaS, respectively. For sample application, a 10 mL Omnifix Luer Lock Solo syringe (B. Braun, Melsungen, Germany) was attached to the respective device. The subsequent wash step involved 4 mL for the Sartobind Q and 2 mL for the PrimaS, utilizing an additional syringe containing a loading and elution buffer mixture with the same gradient elution experiments for MgCl₂ concentration as during sample application. A third syringe was used for isocratic elution with 5 mL at a gradient elution experiments for MgCl₂ concentration of 30.8 mM for the Sartobind Q and 1.5 mL at 9.2 mM gradient elution experiments for MgCl₂ for the PrimaS. A final elution step was performed with a fourth syringe, using 3 mL for the Sartobind Q and 1.5 mL for the PrimaS, each with a gradient elution experiments for MgCl₂ concentration of 50 mM.

### AAV capsid titer determination

AAV capsid titer titration was performed employing the Octet R8 equipped with Octet AAVX Biosensors (both Sartorius). Quantitation was performed at 30°C and a shaking speed of 1000 rpm. The reading time during the quantitation step was set to 900 s. AAV reference standards were procured from Progen Biotechnik (Heidelberg, Germany).

### AAV genome titer determination

AAV viral genome (VG) titers were determined by nanoplate digital polymerase chain reaction (dPCR) using the QIAcuity One with 24-well nanoplates (both Qiagen, Hilden, Germany). The samples were serially diluted in dPCR buffer composed of TE buffer (Thermo Fisher Scientific), 0.01% (v/v) Pluronic F-68 (Sigma-Aldrich, Darmstadt, Germany), and 100 mg/mL Poly A Carrier RNA (Roche, Basel, Switzerland). The diluted samples were then incubated at 95°C for 30 min. The dPCR was performed using the QIAcuity Probe Mastermix (Qiagen) along with specific forward and reverse primers (800 nM) and a specific probe (400 nM) targeting the SV40 polyadenylation signal (Integrated DNA Technologies, Coralville, Iowa).

### AAV2 transducing titer determination

AAV2 transducing unit titers were assessed by live-cell analysis. Adherent HEK293T cells (ACC 635; DSMZ, Braunschweig, Germany) were transduced with AAV2 samples. 4 × 10³ HEK293T cells per well were seeded in DMEM (Thermo Fisher Scientific) supplemented with 10% (v/v) fetal calf serum (FCS) and 0.5% (v/v) penicillin-streptomycin (P/S) (both Sigma-Aldrich). The cells were seeded in a tissue culture treated, poly-L-lysine (Sigma-Aldrich) coated black 96-well plate with a clear bottom (Corning Inc., Corning, NY, USA).

The cells were cultivated for one day at 36.5°C and 5% CO₂ in a static incubator. Prior to the transduction process, the spent culture medium was aspirated, and the cells were transduced by adding 50 µL of AAV2 samples. A medium exchange was performed 24 h post-transduction. The expression of green fluorescent protein (GFP) within the cells was monitored and quantitatively analyzed 48 hpt using the Incucyte^{®} S3 live cell analysis system (Sartorius). Imaging was performed at 10× magnification, utilizing both the phase contrast and the green fluorescence channel at 3 h intervals to assess GFP expression dynamics.

### Western blotting

AAV samples were diluted 6.25:1 in ddH20 and loading dye and subjected to thermal denaturation at 95°C for 3 min. Loading dye was prepared using 900 µL of 4× Laemmli sample buffer and 100 µL of 2-mercaptoethanol, (both Bio-Rad, Hercules, California). A total AAV amount of 2.75 × 10⁹ capsids was loaded per lane on a 10% Mini-Protean TGX gel (Bio-Rad). Electrophoretic separation was conducted at 200 V over 37 min. For western blot analysis, proteins were transferred to a 0.2 µm polyvinylidene fluoride membrane (Trans-Blot Turbo Transfer Pack, Bio-Rad). The membrane was then subjected to a blocking step using a solution of 5% (w/v) bovine serum albumin dissolved in TBS buffer (150 mM NaCl, 3 mM KCl, 25 mM Tris, all Carl Roth) supplemented with 0.1% (v/v) Tween 20 (Sigma-Aldrich) for a duration of 1 h. Following the blocking step, the membrane was incubated for 1 h with a primary antibody specifically targeting the AAV viral protein (VP) VP1/VP2/VP3 (Progen Biotechnik), at a dilution of 1:500 in TBS buffer containing 0.1% (v/v) Tween 20. Post-primary antibody incubation, the membrane was washed with TBS buffer and subsequently incubated with a secondary horseradish peroxidase conjugated anti-mouse antibody (diluted 1:5000 in TBS buffer, Thermo Fisher Scientific). Following a final TBS buffer wash step, the membrane was treated with 400 µL of SuperSignal West Pico Plus chemiluminescence substrate (Thermo Fisher Scientific). The resulting signal was imaged using an UVP ChemStudio imaging system (Analytik Jena, Jena, Germany).

### Example 1: Testing salts for separating full from empty AAV capsids

Two different chromatographic stationary phases (Sartobind Q and PrimaS) were used for separating full AAV from empty AAV capsids. In order to develop a flow-through polishing method for the separation of full AAV from empty AAV capsids, an exemplary cation was selected for effective separation and to determine the conditions under which full AAV capsids still bind to the stationary phase, while empty AAV capsids elute from the column.

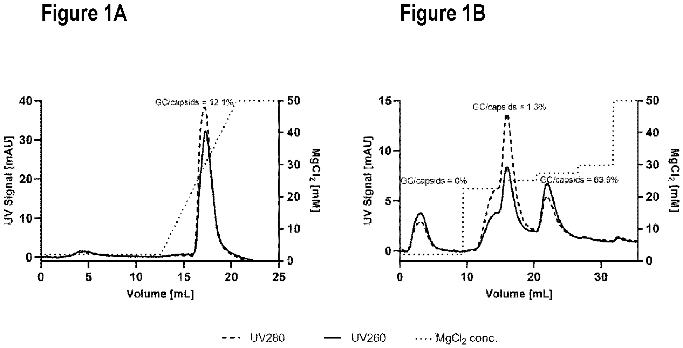

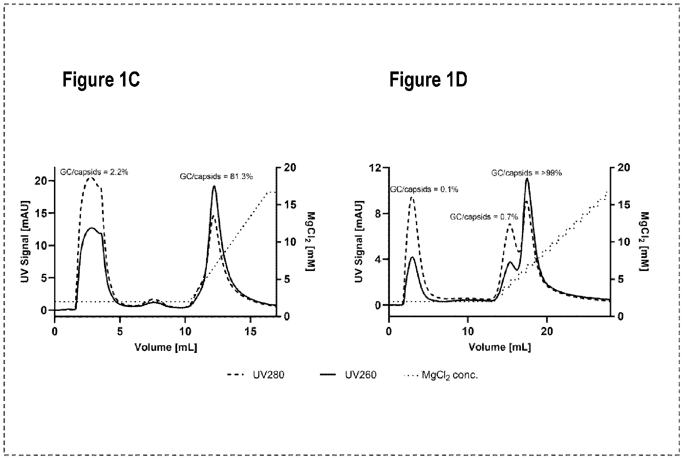

Linear and step gradient elution experiments were performed to identify a suitable cation and the respective concentration, as shown in Figure 1 for AAV8. Given that proteins absorb predominantly at 280 nm and nucleic acids at 260 nm, an increased 260:280 nm absorbance ratio indicates an enrichment of genome-containing AAV capsids. In addition, these results were verified by quantification of capsid and viral genome (VG) titers.

The first stationary phase tested was the Sartobind Lab Q MA. Linear gradient and isocratic elution experiments, as illustrated in Figure 1A and 1B, were performed using an AAV8 sample with a genomic copies (GC)/capsid ratio of 8.4%.

Subsequent elution experiments utilizing MgCl₂ for elution are shown in Figure 1A and B. AAV eluted over a concentration range of 23-38 mM MgCl₂. During the step gradient elution experiment in Figure 1B, a peak devoid of AAV was observed during sample application. Empty AAV capsids with a GC/capsid ratio of 1.3% eluted between 22-25 mM MgCl₂, while full AAV capsids with a GC/capsid ratio of 63.9% eluted between 25-27.5 mM MgCl₂. Due to the separation of the full and empty AAV particles achieved with MgCl₂, it was selected as exemplary salt for flow-through polishing.

Similar linear gradient and isocratic elution experiments were performed with MgCl₂ for AAV5 and AAV2. Based on these findings, the MgCl₂ concentrations for the flow-through polishing were determined to be 24.6 mM for AAV8, 28.6 mM for AAV5, and 15.4 mM for AAV2.

The monolithic PrimaS column was tested as exemplary alternative stationary phase for the chromatographic separation of full AAV capsids from their empty counterparts followed protocols analogous to those used for the Sartobind Q.

Employing MgCl₂ as an elution salt resulted in elution patterns as depicted in Figure 1C and D. It was observed that already during sample application, with a buffer containing 2 mM MgCl₂, mainly empty AAV8 with a GC/capsid ratio of 2.2% were found in the flow-through. The remaining AAV eluted within the range of 2-11 mM MgCl₂. The initial sample used for the step gradient elution experiment shown in Figure 1D had a GC/capsid ratio of 9.5%. The inventors found that in addition to the empty AAV8 in the flow-through, an additional fraction of empty AAV8 was eluted between 3-4 mM MgCl₂ (GC/capsid ratio of 0.7%). Genome-containing AAV8 capsids were subsequently eluted between 4-10 mM MgCl₂. Similar to the observations with the Sartobind Q, MgCl₂ demonstrated good separation of full and empty AAV peaks and was therefore selected as the salt for flow-through polishing.

Comparable linear gradient and isocratic elution experiments with MgCl₂ were performed for AAV5 and AAV2. Based on these results, the MgCl₂ concentration of the samples for the flow-through polishing was adjusted to 3.6 mM for AAV8, 11.6 mM for AAV5, and 0.1 mM for AAV2.

### Example 2: Membrane adsorber-based flow-through separation of full from empty AAV capsids

Flow-through polishing conditions for the Sartobind Q were determined by linear and step gradient elution experiments, as described in example 1.

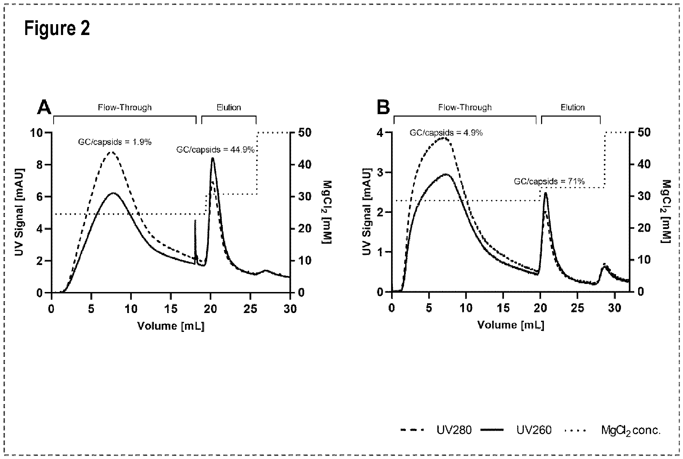

The chromatographic profiles resulting from the flow-through polishing of AAV8, AAV5, and AAV2 are shown in Figure 2A-C. Detailed process parameters are shown in Table 2.

**Table 2: Overview of flow-through polishing process characteristics.**

| | **PrimaS** | | | **Sartobind Q** | | |
|---|---|---|---|---|---|---|
| Serotvpe | AAV8 | AAV5 | AAV2 | AAV8 | AAV5 | AAV2 |
| MgCl₂ [mM] | FT: 3.6 | FT: 11.6 | FT: 0.1 | FT: 24.6 | FT: 28.6 | FT: 15.4 |
| | Elution: 9.2 | Elution: 1.2 | Elution: 3 | Elution: 30.8 | Elution: 32.7 | Elution: 20.2 |
| Final VG recovery [%] | FPLC: 33 | FPLC: 42 | FPLC: 18 | FPLC: 18 | FPLC: 18 | FPLC: 27 |
| | Syringe: 62.9 | | | Syringe: 25.5 | | |
| Full AAV enrichment factor [-] | FPLC: 4.6 (10.4% - 47.5%) | FPLC: 7.5 (10.1% - 76.2%) | FPLC: 1.9 (6.3% - 12.1%) | FPLC: 5.3 (8.4% - 44.9%) | FPLC: 7 (10.1%-71%) | FPLC: 4 (5.9%-23.8%) |
| | Syringe: 3.6 (10.4% - 37.2%) | | | Syringe: 5.4 (9.3% - 49.8%) | | |

Information on required MgCl₂ concentrations, final VG yields in the full AAV elution peaks, and the enrichment factor of the full AAV for the flow-through polishing of AAV8, AAV5, and AAV2 using either a PrimaS column or a Sartobind Q membrane adsorber. Process characteristics are shown for both FPLC-dependent and FPLC-independent ("syringe") flow-through polishing. The full AAV enrichment factor is defined as the increase in the GC/capsid ratio in the full AAV elution peaks compared to the affinity-purified starting material. The corresponding GC/capsid ratios before and after flow-through polishing are given in parentheses. FT, flow-through; VG, viral genome; FPLC, fast protein liquid chromatography; GC, genome copies.

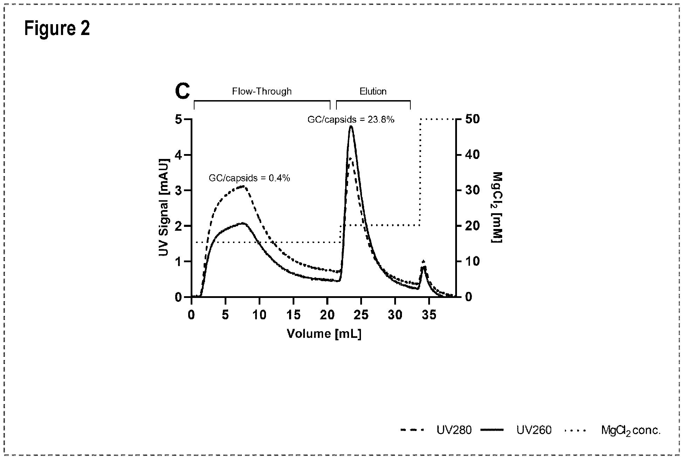

For AAV8 (Figure 2A), the flow-through polishing was performed at a MgCl₂ concentration of 24.8 mM, with elution of the genome-containing AAV8 capsids at 30.8 mM MgCl₂. The flow-through polishing resulted in a 5.3-fold enrichment of the full AAV8 particles, with the GC/capsid ratio increasing from 8.4% in the feed material to 44.9% in the eluted fraction at 30.8 mM MgCl₂. At the same time, the GC/capsid ratio in the flow-through was reduced to 1.9%. Flow-through polishing of AAV5 was achieved by sample application at 28.6 mM MgCl₂, with elution of the genome-containing AAV performed at 32.7 mM MgCl₂ (Figure 2B). The GC/capsid ratio was reduced from 10.1% in the starting material to 4.9% in the flow-through, while the GC/capsid ratio in the elution fraction at 32.7 mM MgCl₂ was increased by a factor of 7 to 71%. For AAV2 (Figure 2C), the flow-through polishing was performed at 15.4 mM MgCl₂, while the genome-containing AAV2 capsids were eluted at 20.2 mM MgCl₂. Flow-through polishing reduced the GC/capsid ratio from 5.9% in the feed material to 0.4% in the flow-through, while increasing it by a factor of 4 to 23.8% in the elution at 20.2 mM MgCl₂.

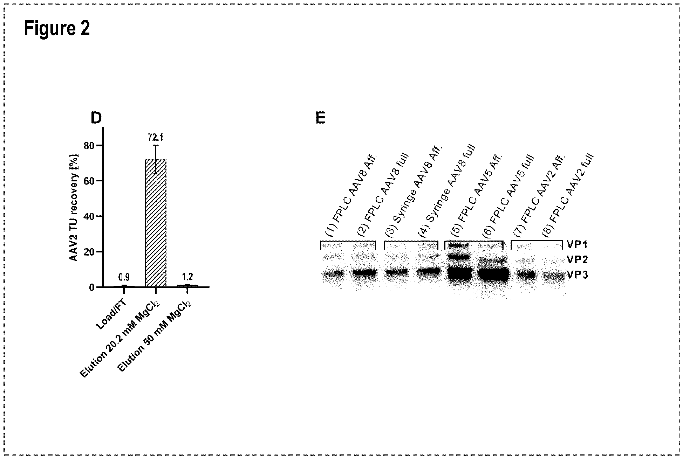

The transduction efficiency of AAV2 following flow-through polishing was quantitatively assessed by the transduction of HEK293T cells, as shown in Figure 2D. The analysis revealed that 0.9% of the total transducing units were found in the flow-through, while the 20.2 mM MgCl₂ elution showed a transducing unit recovery of 72.1%.

Subsequently, the second elution peak at 50 mM MgCl₂ contained 1.2% of the total transducing units. In addition to potency, the integrity of AAV capsids before and after flow-through polishing was analyzed using a western blot targeting VP1, VP2, and VP3 (Figure 2E). The band intensity ratios for VP1, VP2, and VP3 remained constant at a ratio of 1:1:10 both before and after flow-through polishing, indicating no change in the composition of AAV8, AAV5, and AAV2 capsids.

### Example 3: Monolith-based flow-through separation of full from empty AAV capsids

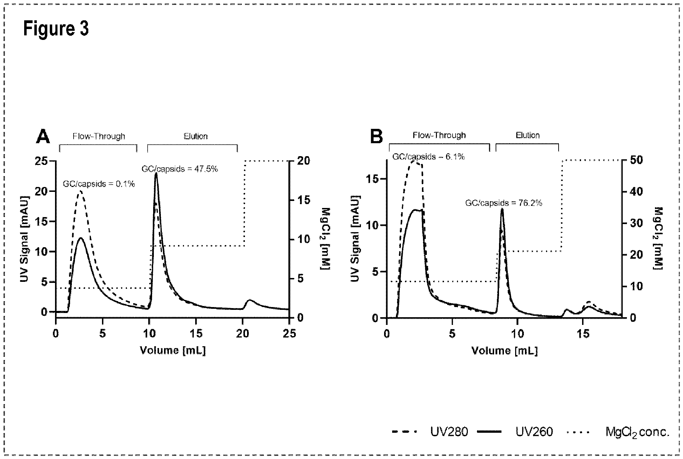

The resulting chromatographic profiles of the PrimaS flow-through polishing (conditions determined in example 1) of AAV8, AAV5, and AAV2 are presented in Figure 3A-C, respectively.

Detailed process characteristics are provided in above Table 2.

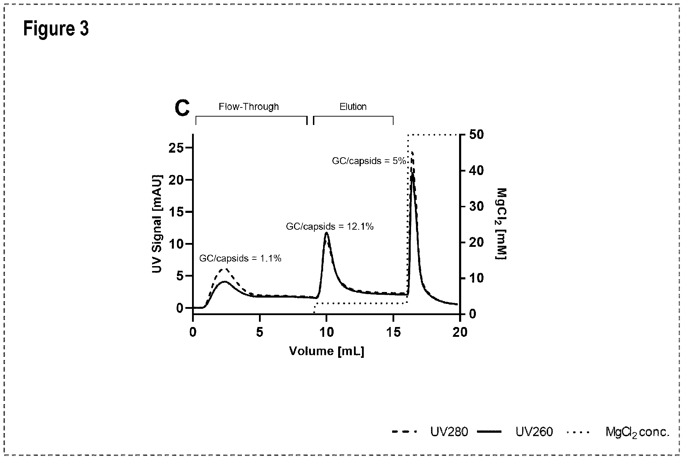

For AAV8 (Figure 3A), the flow-through polishing was conducted at a MgCl₂ concentration of 3.6 mM, with the elution of genome-containing AAV8 capsids at 9.2 mM MgCl₂. This step resulted in an enrichment of the full AAV8 particles by a factor of 4.6 from a GC/capsid ratio of 10.4% in the starting material to 47.5% in the eluted fraction at 9.2 mM MgCl₂. The GC/capsid ratio in the flow-through could be reduced to 0.1%. The flow-through polishing of AAV5, depicted in Figure 3B, was executed at 11.6 mM MgCl₂, while the genome-containing AAV5 capsids were eluted at 21.2 mM MgCl₂. This led to a decrease in the GC/capsid ratio from 10.1% in the feed to 6.1% in the flow-through. In contrast, the elution fraction demonstrated an increase in the GC/capsid ratio to 76.2%, corresponding to an enrichment factor of 7.5 for the genome-containing AAV5 capsids. The flow-through polishing of AAV2 was achieved by sample application at 0.1 mM MgCl₂, with elution of the genome-containing AAV performed at 3 mM MgCl₂. The corresponding chromatographic profile is shown in Figure 3C. While the GC/capsid ratio decreased from 6.3% in the feed material to 1.1% in the flow-through, it increased by a factor of 1.9 to 12.1% in the elution at 3 mM MgCl₂. A second elution peak with a GC/capsid ratio of 5% was observed upon increasing the MgCl₂ concentration to 50 mM, similar to that of the feed material.

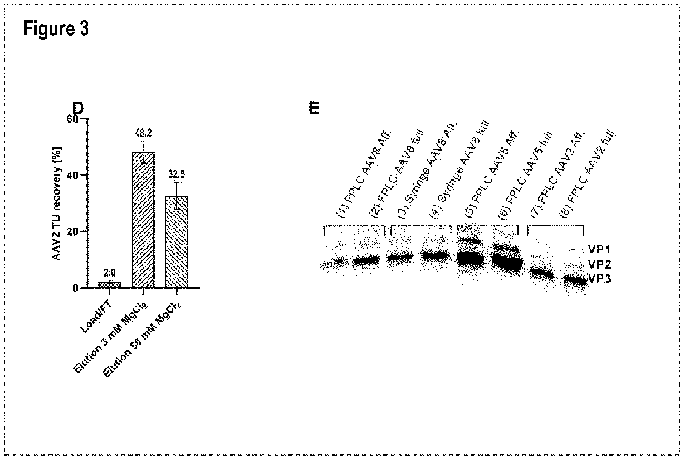

AAV2 transduction efficiency was determined (Figure 3D) with the flow-through comprising 2% of the total transducing units. The elution of full AAV2 particles at 3 mM MgCl₂ resulted in a transducing unit recovery of 48.2%. The second elution peak at 50 mM MgCl₂ contained 32.5% of the total transducing units.

AAV capsid integrity was assessed by western blot analysis, as shown in Figure 3E. Consistent band intensity ratios of 1:1:10 for VP1, VP2, and VP3 were maintained both before and after-flow-through polishing. The capsid composition of AAV remained unchanged throughout the flow-through polishing process.

### Example 4: FPLC-independent flow-through separation of full from empty AAV capsids

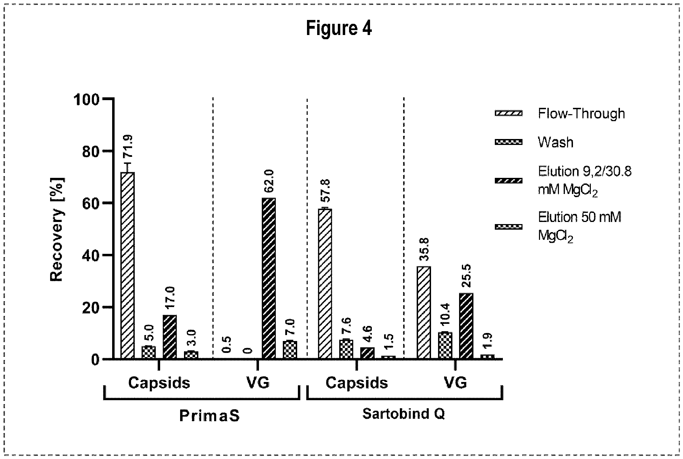

The flow-through polishing conditions established for AAV8 in examples 2 and 3 were adapted to an FPLC-independent system, utilizing separate syringes with varying MgCl₂ concentrations for each individual step (Figures 4 and 5). Details of the experimental setup and process information are provided in above Table 2.

Flow-through polishing was performed at 3.6 mM MgCl₂ for PrimaS and at 24.6 mM MgCl₂ for Sartobind Q, followed by elution using separate syringes with an MgCl₂ concentration of 9.2 mM and 30.8 mM, respectively. Utilizing the PrimaS, 71.1% of the capsids were found in the flow-through, and 17.6% in the elution fraction at 9.2 mM MgCl₂. In contrast, <1% of VG were detected in the flow-through, while 62.9% of the VG were recovered in the elution fraction at 9.2 mM MgCl₂. This resulted in a decrease in the GC/capsid ratio from 10.4% in the feed to 0.1% in the flow-through. Conversely, the elution fraction demonstrated an increase in the GC/capsid ratio to 37.2%, corresponding to an enrichment factor of 3.6 for the genome-containing AAV8 capsids. Manual flow-through polishing of AAV8 with the Sartobind Q yielded a capsid recovery of 57.8% in the flow-through, 7.6% in the wash, and 4.6% in the elution fraction at 30.8 mM MgCl₂. 35.8% of the VG were found in the flow-through, 10.4% in the wash, and 25.5% in the 30.8 mM MgCl₂ elution fraction. The GC/capsid ratio decreased from 9.3% in the starting material to 5.6% in the flow-through, while the elution fraction at 30.8 mM MgCl₂ demonstrated a 5.4-fold increase in the GC/capsid to 49.8% Additionally, the integrity of the AAV Capsid was assessed by western blot analysis and confirmed to be maintained throughout the flow-through polishing process, as evidenced in Figure 2E and Figure 3E.

The efficacy of the method disclosed herein was therefore demonstrated for three exemplary AAV serotypes (AAV8, AAV5, and AAV2) on two exemplary stationary phases: a membrane adsorber and a monolith, resulting in a 4 to 7.5-fold enrichment of full viral particles. Manual flow-through polishing using either the monolith or membrane adsorber achieved 3.6-fold and 5.4-fold enrichment of full capsids, respectively.

The novel method disclosed herein enables separation of full and empty AAV without a pH shift and is therefore user-friendly. The method can be used for the separation of full and empty capsids across multiple serotypes in larger scale processes but also to small scale laboratory settings without the need for FPLC systems.

### Example 5: Steric exclusion chromatography followed by flow-through separation of full from empty AAV capsids

Example 5 was performed using the materials and methods set out below.

### AAV production and harvest

AAV8 were produced via transient transfection of HEK293 cells (Expi293F Inducible Cells, Thermo Fisher Scientific, Waltham, MA) a 10 L glass Univessel^{®} bioreactor (Sartorius, Göttingen, Germany). FreeStyle 293 Expression Medium (Thermo Fisher Scientific) was used as the growth medium. Within the 10 L bioreactor, the cultivation conditions were regulated by the BIOSTAT^{®} DCU controller unit (Sartorius) to maintain set points of 201 rpm, 40% dissolved oxygen, and pH 7.25. Furthermore, to prevent foaming within the bioreactor, the cultivation medium within the bioreactor was supplemented with antifoam C (Sigma Aldrich, Darmstadt, Germany) at a final concentration of 0.0002% (v/v). AAV production processes were inoculated at 3 × 10⁵ viable cells/mL. After reaching a cell density of 1.3 × 10⁶ viable cells/mL, transfection was performed using a dual plasmid system, with 0.55 µg/mL DNA per 10⁶ viable cells and FectoVIR AAV (Polyplus, Illkirch, France) as the transfection reagent in a 1:1 ratio. The plasmids pAAV ssGFP (vector plasmid) and pDP8.ape (packaging and helper plasmid) were used for transfection in a molar ratio of 4.5:1. Plasmids were sourced from PlasmidFactory (Bielefeld, Germany). At 72 h post transfection, AAV vectors were harvested. To initiate cell lysis, the cell broth was treated with a 20x lysis buffer (1 M Tris pH 8.0, 40 mM MgCl₂ (both Carl Roth, Karlsruhe, Germany), 20% (v/v) Tween 20 (Sigma Aldrich)) and Denarase (c Lecta, Leipzig, Germany) at a final concentration of 50 U/mL. The mixture was then incubated at 37°C for 1.5 h. Subsequently, the lysed cultures were treated with 500 mM NaCl and incubated at 37°C for another 30 min.

### Purification of AAV from crude cell lysates by steric exclusion chromatography

Clarification of AAV crude cell lysates was performed by alluvial filtration as previously reported. Briefly, the crude cell lysate was mixed with 11.25 g/L of Sartoclear Dynamics^{®} Lab Filter Aid (Sartorius) and clarified using Sartolab^{®} RF1000 filters (0.22 µm; PES; Sartorius). The clarified AAV sample was kept on ice and further purified by Steric Exclusion Chromatography SXC using hydrophilic membrane adsorber (MA) prototypes (Sartorius, Göttingen, Germany) and an ÄKTA^{™} avant 25 (Cytiva, Marlborough, MA, USA) chromatography system. Four distinct buffers were prepared as follows: (A) 10 mM Bis-Tris Propane pH 8.5, 2 mM MgCl₂, 72.5/74.75 mM NaCl, 1% (w/v) D(+)-Saccharose, 0.1% (w/v) Kolliphor P188, (B) 10 mM Bis-Tris Propane pH 8.5, 2 mM MgCl₂, 72.5/74.75 mM NaCl, 1% (w/v) D(+)-Saccharose, 0.1% (w/v) Kolliphor P188, 40% (w/v) PEG2000, and (C) 100 mM Bis-Tris Propane pH 8.5, 20 mM MgCl₂, 725/747.5 mM NaCl, 10% (w/v) D(+)-Saccharose, 1% (w/v) Kolliphor P188, hereafter referred to as PEG free (A), PEG buffer (B), and 10x PEG free buffer (C). All chemicals were purchased from Carl Roth and Sigma Aldrich. Clarified AAV material was pre conditioned with 10x PEG free buffer. The MA was first equilibrated with 20 membrane volumes (MV) of PEG free buffer. Subsequently, a total of 30 mL of preconditioned clarified AAV sample was loaded per run in a downflow direction onto the column at a flow rate of 5.35 mL/min and a final PEG2000 concentration of 15% by mixing it with PEG buffer in the respective ratio. Following sample application, the column was washed with 25 MV at a final PEG2000 concentration of 15% by mixing PEG free buffer and PEG buffer in the respective ratio. The AAV were then eluted with PEG free buffer in an upflow direction. The first 2 mL of the elution fraction were discarded as they did not contain any AAV. Elution fractions were stored at 80°C until further use.

### Anion exchange flow through separation of full from empty AAV capsids

For the flow through separation of full from empty AAV capsids, the monolithic column CIMmultus/CIMmic QA (2 µm pore size, 1 mL, and 0.1 mL, Sartorius BIA Separations, Ajdovscina, Slovenia) was used. Two different buffers were prepared as follows: (A) 10 mM Bis-Tris Propane pH 8.5, 2 mM MgCl₂, 72.5/4.75 mM NaCl, 1% (w/v) D(+)-Saccharose, 0.1% (w/v) Kolliphor P188, and (B) 10 mM Bis-Tris Propane pH 8.5, 2 mM MgCl₂, 500 mM NaCl, 1% (w/v) D(+)-Saccharose, 0.1% (w/v) Kolliphor P188, hereafter referred to as loading and elution buffer. Stationary phase was cleaned and regenerated based on manufacturer's recommendations. Experiments were conducted on an ÄKTA^{™} avant 25 (Cytiva) chromatography system at a flow rate of 1 mL/min. All chemicals were purchased from Carl Roth and Sigma Aldrich.

For the NaCl flow through polishing approach, 8 × 10¹² capsids of SXC purified AAV8 were loaded onto the column using a 10 mL sample loop, followed by a washing step with the loading buffer for 5 8 mL at a final concentration 72.5/74.75 mM NaCl. Isocratic elution of full AAV particles was then achieved by increasing the NaCl concentration to 123.8 mM by mixing the loading and elution buffer in the respective ratio. Further elution of impurities was performed by increasing the NaCl concentration to 253 mM and 500 mM NaCl.

### AAV capsid titer determination

AAV capsid titer titration was performed employing the Octet R8 equipped with Octet AAVX Biosensors (both Sartorius). Quantitation was performed at 30°C and a shaking speed of 1000 rpm. The reading time during the quantitation step was set to 900 s. AAV reference standards were procured from Progen Biotechnik (Heidelberg, Germany).

### AAV genome titer determination

AAV VG titers were determined by nanoplate digital polymerase chain reaction (dPCR) using the QIAcuity One with 24 well nanoplates (both Qiagen, Hilden, Germany). The samples were serially diluted in dPCR buffer composed of TE buffer (Thermo Fisher Scientific), 0.01% (v/v) Pluronic F 68 (Sigma Aldrich, Darmstadt, Germany), and 100 mg/mL Poly A Carrier RNA (Roche, Basel, Switzerland). The diluted samples were then incubated at 95°C for 30 min. The dPCR was performed using the QIAcuity Probe Mastermix (Qiagen) along with specific forward and reverse primers (800 nM) and a specific probe (400 nM) targeting the SV40 polyadenylation signal (Integrated DNA Technologies, Coralville, Iowa).

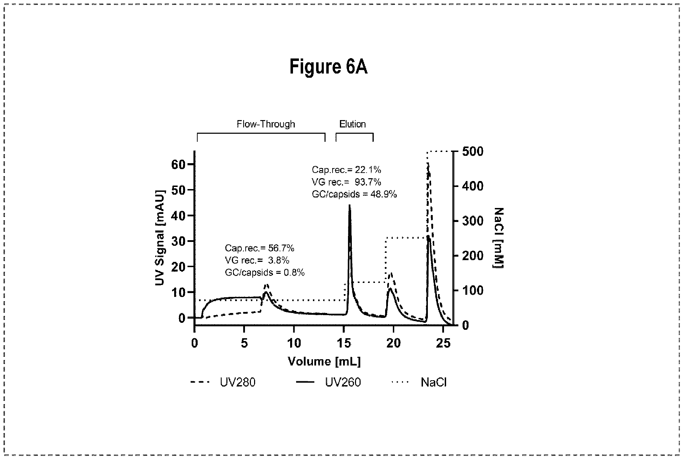

Results are shown in fig. 6A (flow-through polishing of AA8 at a final NaCl of 72.5 mM) and 6B (flow-through polishing of AAV8 at a final NaCl of 74.75 mM). Following purification by Steric Exclusion Chromatography SXC as described above and without further adapting any of the viral capsid preparation components, the flow-through polishing was performed. The flow-through polishing resulted in an enrichment of the full AAV8 particles, with the GC/capsid ratio increasing to 48.6% or 48.9% in the eluted fraction at 123 mM NaCl. At the same time, the GC/capsid ratio in the flow-through was reduced to 0.8%.

The novel method disclosed herein enables separation of full and empty AAV without the need to adjust the components, component concentration, and pH following purification of crude cell harvests.

### BRIEF DESCRIPTION OF THE DRAWINGS

Figure 1 depicts chromatographic profiles of linear and isocratic elution for the separation of full and empty AAV8 capsids. Panels A+B show the linear and isocratic MgCl₂ elution profiles using Sartobind Q. In addition, panels C+D illustrate the linear and isocratic MgCl₂ elution profiles using PrimaS. GC, genomic copies.
Figure 2 depicts chromatographic profiles of Sartobind Q-based flow-through separation of full and empty AAV capsids. Panels A, B, and C illustrate the flow-through polishing for the separation of full from empty AAV capsids for AAV8, AAV5, and AAV2, respectively. The MgCl₂ concentration during sample application was adjusted to 24.6 mM for AAV8, 28.6 mM for AAV5, and 15.4 mM for AAV2. Panel D provides information on the potency (n=3) of AAV2 following the flow-through polishing. Additionally, panel E shows a western blot analysis, using a monoclonal antibody against the AAV capsid proteins VP1-3, to validate the integrity of the capsid proteins after flow-through polishing. Lanes 1, 3, 5, and 7 represent affinity-purified AAV material, while lanes 2, 4, 6, and 8 correspond to the respective full AAV elution peaks. GC, genomic copies; TU, transducing unit; FT, flow-through; FPLC, fast protein liquid chromatography; Aff., affinity-purified AAV material; VP, viral protein.
Figure 3 depicts chromatographic profiles of PrimaS-based flow-through separation of full and empty AAV capsids. Panels A, B, and C show the flow-through polishing for the separation of full from empty AAV capsids for AAV8, AAV5, and AAV2, respectively. The MgCl₂ concentration during sample application was adjusted to 3.6 mM for AAV8, 11.6 mM for AAV5, and 0.1 mM for AAV2. Panel D shows the potency (n=3) of AAV2 following the flow-through polishing. Additionally, a western blot analysis is shown in panel E, using a monoclonal antibody against the AAV capsid proteins VP1-3, to validate the integrity of the capsid proteins post-flow-through polishing. Affinity-purified AAV material is represented in lanes 1, 3, 5, and 7, while the corresponding full AAV elution peaks are shown in lanes 2, 4, 6, and 8. GC, genomic copies; TU, transducing unit; FT, flow-through; FPLC, fast protein liquid chromatography; Aff., affinity-purified AAV material; VP, viral protein.
Figure 4 depicts the syringe-driven flow-through polishing of AAV8 as exemplary virus. Flow-through polishing of AAV8 was performed using either a PrimaS column or a Sartobind Q membrane adsorber. During sample application, the MgCl₂ concentration was adjusted to 3.6 mM for the PrimaS and 24.6 mM for the Sartobind Q. Elution of full AAV8 was achieved by using a separate syringe with a MgCl₂ concentration of 9.2 mM for the PrimaS and 30.8 mM for the Sartobind Q. A final elution was performed with 50 mM MgCl₂. VG, viral genomes.
Figure 5 depicts exemplary syringe-based systems. Panel A depicts CIMmultus PrimaS^{®} and panel B depicts Sartobind Q^{®}. The FPLC-independent flow-through polishing of AAV8 was performed using several syringes with different MgCl₂ concentrations connected to a PrimaS column or a Sartobind Q membrane adsorber. During sample application, the MgCl₂ concentration was adjusted to 24.6 mM for the Sartobind Q and 3.6 mM for the PrimaS. Elution of full AAV8 was achieved by using a separate syringe with a MgCl₂ concentration of 30.8 mM for the Sartobind Q and 9.2 mM for the PrimaS.

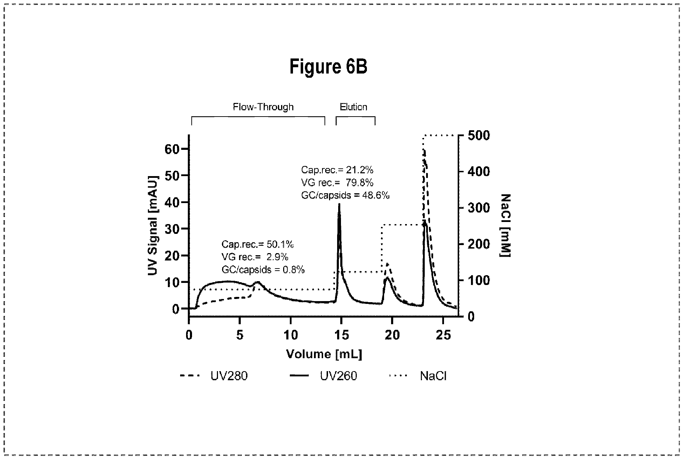

Figure 6 depicts the syringe-driven flow-through polishing of AAV8 as exemplary virus following purification of crude cell lysates without prior adjustment of the salt concentration and pH value. Flowthrough was performed using 72.5 mM or 74.75 mM. NaCl elution of full AAV8 was achieved by using a FPLC system with a NaCl concentration of 123.8 mM.

## Claims

1. A method of separating empty viral capsids and full viral capsids, the method comprising:
(i) providing a viral capsid preparation comprising the empty viral capsids and the full viral capsids, wherein the viral capsid preparation comprises a first salt concentration;
(ii) contacting the viral capsid preparation with a stationary phase surface allowing binding of the full viral capsids to the stationary phase surface, wherein the empty viral capsids at least partially do not bind to the stationary phase surface; and
(iii) conducting a one-step elution and/or linear salt gradient elution in which the full capsids are eluted by contacting the stationary phase obtained in step (ii) with an elution solution comprising a second salt concentration.
wherein the first salt concentration is lower than the second salt concentration.

2. The method according to claim 1, wherein the one-step elution is an isocratic elution.

3. The method according to any of the preceding claims, wherein the pH of the viral capsid preparation and the pH of the elution solution are within a range of about 6 to about 10, preferably about 7 to about 9, preferably about 8 to about 9.

4. The method according to any of the preceding claims, wherein the salt comprises or is selected from magnesium sulfate, sodium sulfate, calcium sulfate, magnesium chloride, sodium chloride, calcium chloride, magnesium acetate, sodium acetate, calcium acetate, or any combination thereof.

5. The method according to any of the preceding claims, wherein the full viral capsids and empty viral capsids are *Adeno-associated viruses* (AAV) capsids.

6. The method according to any of the preceding claims, wherein the method comprises a single contacting step (ii) and/or elution step (iii).

7. The method according to any of the preceding claims, wherein the viral capsid preparation and/or elution solution comprises a buffering component controlling the pH at about 6 to about 10, preferably about 7 to about 9, preferably about 8 to about 9.

8. The method according to any of the preceding claims, wherein the first salt concentration is in the range of 0.01 to 100 mM.

9. The method according to any of the preceding claims, wherein the second salt concentration is in the range of 0.1 to 140 mM.

10. The method according to any of the preceding claims, wherein the full viral capsids and empty viral capsids are AAV2 capsids, optionally wherein the first salt concentration is in the range of about 0.01 to about 10 mM, about 0.02 to about 8 mM, about 0.03 to about 7 mM, about 0.04 to about 6 mM, about 0.05 to about 5 mM, about 0.06 to about 4 mM, about 0.07 to about 3 mM, or about 0.08 to about 2 mM, and/or wherein the second salt concentration is in the range of about 0.1 to about 15 mM, about 0.5 to about 13 mM, about 1 to about 11 mM, about 1.5 to about 9 mM, about 2 to about 7 mM, about 2.5 to about 5 mM, or about 3 to about 4 mM.

11. The method according to any of claims 1-9, wherein the full viral capsids and empty viral capsids are AAV5 capsids, optionally wherein the first salt concentration is in the range of about 0.5 to about 100 mM, about 1 to about 80 mM, about 2 to about 60 mM, about 3 to about 40 mM, about 4 to about 30 mM, about 5 to about 25 mM, about 6 to about 20 mM, or about 7 to about 15 mM, and/or wherein the second salt concentration is in the range of about 1 to about 140 mM, about 3 to about 120 mM, about 4 to about 100 mM, about 5 to about 80 mM, about 8 to about 60 mM, about 9 to about 40 mM, or about 10 to about 30 mM.

12. The method according to any of claims 1-9, wherein the full viral capsids and empty viral capsids are AAV8 capsids, optionally wherein the first salt concentration is in the range of about 0.1 to about 100 mM, about 0.5 to about 80 mM, about 1 to about 60 mM, about 1.5 to about 40 mM, about 2 to about 30 mM, about 2.5 to about 20 mM, or about 3 to about 10 mM, and/or wherein the second salt concentration is in the range of about 1 to about 140 mM, about 2 to about 120 mM, about 3 to about 100 mM, about 4 to about 80 mM, about 5 to about 60 mM, about 6 to about 40 mM, or about 7 to about 20 mM.

13. The method according to any of the preceding claims, wherein the method further comprises the step of providing a crude cell harvest or crude cell lysate comprising the first salt concentration, and purifying the crude cell harvest or crude cell lysate to obtain the viral capsid preparation of step (i).

14. The method according to any of the preceding claims, wherein the method is conducted in a syringe-based system.

15. A kit comprising:
(i) a first container comprising a preparation solution with a first salt concentration
(ii) a second container comprising an elution solution with a second salt concentration
wherein the first salt concentration is lower than the second salt concentration.
